# EUROPEAN PATENT SPECIFICATION

(11) **EP 4 054 438 B1**
(45) Date of publication and mention of the grant of the patent: **11.03.2026**
(21) Application number: 20816073.9
(22) Date of filing: 03.11.2020
(51) Int. Cl.: A61B 17/12

(54) **DEVICES, SYSTEMS, AND METHODS FOR TREATMENT OF INTRACRANIAL ANEURYSMS**
VORRICHTUNGEN, SYSTEME UND VERFAHREN ZUR BEHANDLUNG VON INTRAKRANIELLEN ANEURYSMEN
DISPOSITIFS, SYSTÈMES ET PROCÉDÉS POUR LE TRAITEMENT D'ANÉVRISMES INTRACRÂNIENS

(30) Priority: 04.11.2019 US 201962930303 P; 04.11.2019 US 201962930487 P; 04.11.2019 US 201962930421 P; 04.11.2019 US 201962930333 P; 04.11.2019 US 201962930357 P; 04.11.2019 US 201962930324 P
(43) Date of publication of application: 14.09.2022
(73) Proprietor: Covidien LP, Mansfield, MA 02048 (US)
(72) Inventor: LI, Junwei, Irvine, CA 92620 (US); WAINWRIGHT, John, Foothill Ranch, CA 92610 (US); GIRDHAR, Gaurav, Ladera Ranch, CA 92694 (US); SOSNOWSKI, Stephen, Sherrills Ford, NC 28673 (US); RHEE, Richard, Anaheim, CA 92808 (US); DIVINO, Vincent, Mission Viejo, CA 92692 (US); NAGESWARAN, Ashok, Irvine, CA 92606 (US); ASHBY, Mark, Laguna Niguel, CA 92677 (US); NGUYEN, Hoai, Westminster, CA 92683 (US); PATEL, Gopan, Orange, CA 92869 (US); BROWN, Kenneth, Oceanside, CA 92057 (US); SKILLRUD, Erik, Irvine, CA 92612 (US); PULUGURTHA, Syamala, Rani, Irvine, CA 92618 (US); JALGAONKAR, Ujwal, Irvine, CA 92620 (US); MINTZ, Eric, Newport Coast, CA 92657 (US); HUYNH, Andyanhdzung, Westminster, CA 92683 (US)
(74) Representative: Gray, James
(86) International application number: PCT/US2020/070741
(87) International publication number: WO 2021/092618

(56) References cited:
- WO-A1-2015/166013
- WO-A1-2019/038293
- CA-A1- 3 031 482
- US-A1- 2007 198 075
- US-A1- 2017 156 734
- US-A1- 2017 367 707
- US-A1- 2018 070 955
- US-A1- 2018 110 797
- US-B2- 6 723 112

## Description

### CROSS-REFERENCE TO RELATED APPLICATIONS

The present application claims the benefit of priority of U.S. Provisional Application No. 62/930,421, filed November 4, 2019, U.S. Provisional Application No. 62/930,487, filed November 4, 2019, U.S. Provisional Application No. 62/930,303, filed November 4, 2019, U.S. Provisional Application No. 62/930,324, filed November 4, 2019, U.S. Provisional Application No. 62/930,333, filed November 4, 2019, and U.S. Provisional Application No. 62/930,357, filed November 4, 2019.

### TECHNICAL FIELD

The present technology relates to systems, devices, and methods for treating intracranial aneurysms.

### BACKGROUND

An intracranial aneurysm is a portion of an intracranial blood vessel that bulges outward from the blood vessel's main channel. This condition often occurs at a portion of a blood vessel that is abnormally weak because of a congenital anomaly, trauma, high blood pressure, or for another reason. Once an intracranial aneurysm forms, there is a significant risk that the aneurysm will eventually rupture and cause a medical emergency with a high risk of mortality due to hemorrhaging. When an unruptured intracranial aneurysm is detected or when a patient survives an initial rupture of an intracranial aneurysm, vascular surgery is often indicated. One conventional type of vascular surgery for treating an intracranial aneurysm includes using a microcatheter to dispose a platinum coil within an interior volume of the aneurysm. Over time, the presence of the coil should induce formation of a thrombus. Ideally, the aneurysm's neck closes at the site of the thrombus and is replaced with new endothelial tissue. Blood then bypasses the aneurysm, thereby reducing the risk of aneurysm rupture (or re-rupture) and associated hemorrhaging. Unfortunately, long-term recanalization (i.e., restoration of blood flow to the interior volume of the aneurysm) after this type of vascular surgery occurs in a number of cases, especially for intracranial aneurysms with relatively wide necks and/or relatively large interior volumes.

Another conventional type of vascular surgery for treating an intracranial aneurysm includes deploying a flow diverter within the associated intracranial blood vessel. The flow diverter is often a mesh tube that causes blood to preferentially flow along a main channel of the blood vessel while blood within the aneurysm stagnates. The stagnant blood within the aneurysm should eventually form a thrombus that leads to closure of the aneurysm's neck and to growth of new endothelial tissue, as with the platinum coil treatment. One significant drawback of flow diverters is that it may take weeks or months to form aneurysmal thrombus and significantly longer for the aneurysm neck to be covered with endothelial cells for full effect. This delay may be unacceptable when risk of aneurysm rupture (or re-rupture) is high. Moreover, flow diverters typically require antiplatelet therapy to prevent a thrombus from forming within the main channel of the blood vessel at the site of the flow diverter. Antiplatelet therapy may be contraindicated shortly after an initial aneurysm rupture has occurred because risk of re-rupture at this time is high and antiplatelet therapy tends to exacerbate intracranial hemorrhaging if re-rupture occurs. For these and other reasons, there is a need for innovation in the treatment of intracranial aneurysms. Given the severity of this condition, innovation in this field has immediate life-saving potential.
US2018070955A1 describes intravascular therapeutic treatment and the use of medical devices and occlusive or embolic material to treat a vascular condition.
US2017/367707 A1 describes detachment of an implant from a delivery assembly can be electrolytic and provide an electrical current pathway through a fluid within a portion of a delivery system containing the delivery assembly.
US6723112 B2 describes an implantable medical device for at least partially obstructing a neck portion of a vascular aneurysm.

### SUMMARY

The invention is defined in independent claim 1. Certain optional features of the invention are defined in the dependent claims.

According to a first aspect of the invention, there is provided a treatment system comprising: an electrolytically corrodible core wire having a proximal portion, a distal portion, and a detachment zone between the proximal portion and the distal portion; an occlusive member having a proximal hub coupled to the core wire distal portion, the occlusive member configured to be positioned at or adjacent to a treatment site; and a conduit extending along at least a portion of the core wire, the conduit having a lumen configured to pass an embolic element therethrough. A distal portion of conduit is configured to be disposed at or adjacent the treatment site alongside the occlusive member. The treatment site comprises an aneurysm sac, and the occlusive member is configured to be disposed within the aneurysm sac and a distal portion of the conduit is configured to be disposed within the aneurysm sac. for delivery of the embolic element to within the aneurysm sac.

Optionally, the conduit comprises a flexible tubular member.

Optionally the treatment system further comprises a stylet configured to be removably disposed within the lumen of the conduit.

Optionally, the stylet is more rigid than the conduit.

Optionally, the stylet is metallic.

Optionally, the conduit has a distal portion having a smaller cross-sectional dimension than a proximal portion of the conduit.

Optionally, the stylet has a distal portion having a smaller cross-sectional dimension than a proximal portion of the stylet.

Optionally, the conduit is coupled to the elongated member such that the two cannot slide relative to one another.

Optionally, the conduit is coupled to the elongated member via one or more bands or clamps.

Optionally, the one or more bands or clamps circumferentially surround both the conduit and the core wire.

Optionally, the conduit is coupled to the elongated member via an adhesive.

Optionally, the conduit is coupled to the elongated member via a surrounding sheath.

Optionally, the surrounding sheath comprises a heat-shrink polymer.

Optionally, the heat-shrink polymer comprises PTFE.

Optionally, the conduit comprises a tubular member, a hypotube, and/or a catheter.

Optionally, the conduit comprises a liner extending through a lumen of a tubular member, the liner extending distally beyond a distal end of the tubular member.

Optionally, the liner comprises extruded PTFE.

Optionally, the conduit has an inner diameter along at least a portion of its length of between 0.127 mm (0.005 inches) and 0.381 mm (0.015 inches).

Optionally, the core wire comprises a proximal insulating layer annularly contacting the proximal portion of the core wire and a distal insulating layer annularly contacting the distal portion of the core wire.

Optionally, the detachment zone has a microstructure with lower crystallinity than proximal and distal portions of the core wire.

Optionally, the detachment zone has a microstructure that is more amorphous than each of the core wire proximal portion and the core wire distal portion.

Optionally, the core wire is made of an electrically conductive material.

Optionally, the detachment zone has an axial length that is less than 0.254 mm (0.010 inches).

Optionally, the detachment zone has an axial length greater than or equal to 0.127 mm (0.005 inches) and less than 0.254 mm (0.010 inches).

Optionally, the core wire comprises an anchor end that is distal to a distalmost end of the hub, the anchor end having a maximum cross-sectional dimension that is greater than an inner cross-sectional dimension of a lumen of the hub.

Optionally, detachment zone is axially between the core wire proximal portion and the core wire distal portion
Optionally, the occlusive member is an occlusive member or intrasaccular device configured to be implanted within an aneurysm.

Optionally, the occlusive member comprises an expandable mesh having a constrained state for delivery to an aneurysm and an expanded state in which at least a portion of the mesh is configured to be disposed across a neck of the aneurysm.

Optionally, the expandable mesh comprises a plurality of braided filaments that assume a pre-set, three-dimensional shape in the expanded state.

Optionally, the expandable mesh comprises a braid formed of 24, 32, 36, 48, 64, or 72 filaments.

Optionally, the expandable mesh comprises a braid formed of a plurality of wires, some or all of which have a diameter of at least 0.025 mm (0.001 inches).

Optionally, the expandable mesh comprises a braid formed of a plurality of wires, some or all of which have the same diameter.

Optionally, the expandable mesh comprises a braid formed of a plurality of wires, at least some of which have different diameters.

Optionally, in the expanded state, the expandable mesh forms one of a sphere, a prolate spheroid, or an oblate spheroid.

Optionally, the expandable mesh comprises an inner layer and an outer layer.

Optionally, the expandable mesh has a maximum cross-sectional dimension of 3.0 mm, 3.5 mm, 4.0 mm, 4.5 mm, 5.0 mm, 5.5 mm, 6.0 mm, 6.5 mm, 7.0 mm, 7.5 mm, or 8.0 mm.

Optionally, the expandable mesh is a laser-cut tube.

Optionally, the expandable mesh comprises a plurality of interwoven filaments.

Optionally, the occlusive member is curved along at least a majority of its entire length.

Optionally, wherein the occlusive member is collapsible when contacted by an embolic element.

Optionally, the occlusive member is collapsible when contacted by a synthetic gel or fluid.

Optionally, the occlusive member is configured to rotate about the conduit.

Optionally, the occlusive member is rotatably and slidably coupled to the conduit.

Optionally, the occlusive member has an aperture at a distal portion thereof, and wherein the conduit extends through the aperture.

Optionally, the occlusive member is configured to move axially along the elongated member.

Optionally, the embolic element is a liquid embolic.

Optionally, the embolic element comprises a biopolymer and/or a chemical crosslinking agent.

Optionally, the biopolymer includes chitosan, a derivative of chitosan, an analog of chitosan, or a combination thereof.

Optionally, the chemical crosslinking agent includes genipin, a derivative of genipin, an analog of genipin, or a combination thereof.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1A shows a perspective view of a system for treating an aneurysm in accordance with the present technology.
FIG. 1B shows an enlarged view of a distal portion of the treatment system of FIG. 1A in accordance with the present technology.
FIGS. 1C and 1D are sectioned views of occlusive members in an expanded state in accordance with the present technology.
FIG. 2 shows an embolic kit according to the present technology.
FIGS. 3A-3G depict an example method of treating an aneurysm with the treatment systems of the present technology.
FIGS. 4A-5B show various types of images that may be employed to confirm and/or monitor deployment of the treatment system of the present technology.
FIG. 6 shows a system for treating an aneurysm in accordance with the present technology.
FIGS. 7A-7J depict an example method of treating an aneurysm with the treatment systems of the present technology.
FIG. 8A shows a schematic side view of a treatment system in accordance with aspects of the present technology.
FIG. 8B shows a schematic side cross-sectional view of a portion of the treatment system of FIG. 8A.
FIGS. 9A-9C illustrate delivery of an occlusive member and embolic element to a treatment site in accordance with aspects of the present technology.
FIG. 10 shows a schematic side view of another embodiment of a treatment system in accordance with aspects of the present technology.
FIG. 11A shows a schematic side view of a treatment system in accordance with aspects of the present technology.
FIG. 11B shows a schematic side cross-sectional view of the treatment system shown in FIG. 11A.
FIG. 11C shows a schematic side cross-sectional view of the treatment system of FIG. 11B after electrolytic detachment.
FIG. 12 shows a schematic side cross-sectional view of a portion of another embodiment of a treatment system.
FIG. 13 shows a schematic side cross-sectional view of a portion of another embodiment of a treatment system.
FIG. 14 shows a schematic side cross-sectional view of a portion of another embodiment of a treatment system.
FIGS. 15A-C illustrate delivery of an occlusive member and embolic element to a treatment site in accordance with aspects of the present technology.

### DETAILED DESCRIPTION

Methods for treating intracranial aneurysms in accordance with at least some embodiments of the present technology include positioning an expandable occlusive member within the aneurysm and introducing an embolic element between the occlusive member and an aneurysm wall. Introduction of the embolic element both fills space within the aneurysm cavity and deforms the occlusive member from a first expanded state to a second expanded state to fortify the occlusive member at the neck of the aneurysm. Deformation of the occlusive member from a first expanded state to a second expanded state provides the additional advantage of giving visual confirmation to the physician that the delivered amount of embolic element sufficiently fills the aneurysm cavity. In addition to providing a structural support and anchor for the embolic element, the occlusive member provides a scaffold for tissue remodeling and diverts blood flow from the aneurysm. Moreover, the embolic element exerts a substantially uniform pressure on the occlusive member towards the neck of the aneurysm, thereby pressing the portions of the occlusive member positioned adjacent the neck against the inner surface of the aneurysm wall such that the occlusive member forms a complete and stable seal at the neck.

Once the occlusive member has deployed within the aneurysm and the embolic element has been delivered, the occlusive member may be detached from the delivery assembly. Suitable detachment mechanisms must be as small as possible so as to be guided through the fine bore of the catheter to the treatment site, while on the other hand they must securely and reliably produce detachment of the intrasaccular implant. Absent a reliable detachment of the intrasaccular implant, withdrawal of the core wire and catheter may cause unintended removal of the occlusive member from the cavity to be occluded and thus injure and/or rupture of the wall of the cavity or vessel. In some embodiments, an electrolytic detachment mechanism as described herein can be used to facilitate reliable, controlled detachment of the occlusive member.

The occlusive member can be implanted in body cavities or blood vessels. In addition to the occlusive member, the treatment system can comprise a voltage source, a cathode, a delivery conduit, and a catheter. The occlusive member and the delivery conduit can be coupled together such that both can be slid in the catheter in the longitudinal direction. In some embodiments, a core wire may engage the occlusive member and be adapted to serve as an anode, such that a portion of the core wire is designed to be electrolytically corroded at one or more points so that while in contact with a body fluid, one or more portions of the occlusive member may be released from the core wire. The delivery conduit can be configured to run adjacent to the core wire along its length. The delivery conduit can be configured to pass one or more embolic elements therethrough for intrasaccular delivery. In some embodiments, a stylet can be removably disposed within the conduit to provide for enhanced rigidity and pushability of the conduit while it is being advanced to the treatment site. Once the stylet is removed, the embolic element may be passed through the conduit and delivered to the treatment site. Once the occlusive member and any embolic elements are deployed, current can be applied to the core wire to electrolytically corrode the core wire at a detachment zone. After the core wire has been severed at the detachment zone, the core wire and conduit can be retracted, and the occlusive member may remain in position at the treatment site.

In some embodiments, the occlusive member can be coupled to a distal portion of the conduit, and the conduit can include a detachment zone configured to be electrolytically severed. In various examples, the conduit can be adapted to serve as an anode, such that a portion of the conduit is designed to be electrolytically corroded at one or more points so that while in contact with a body fluid, and the occlusive member may be released from the conduit. The delivery conduit can be configured to pass one or more embolic elements therethrough for intrasaccular delivery. The embolic element may be passed through the conduit and delivered to the treatment site. Once the occlusive member and any embolic elements are deployed, current can be applied to the conduit to electrolytically corrode the conduit at the detachment zone. After the conduit has been severed at the detachment zone, the conduit can be retracted, and the occlusive member may remain in position at the treatment site. In some embodiments, an inner liner and/or an outer sheath extend along at least a portion of the length of the conduit. The outer sheath can include a gap or opening that is aligned with the detachment zone such that the detachment zone of the conduit is exposed to bodily fluids while at the treatment site.

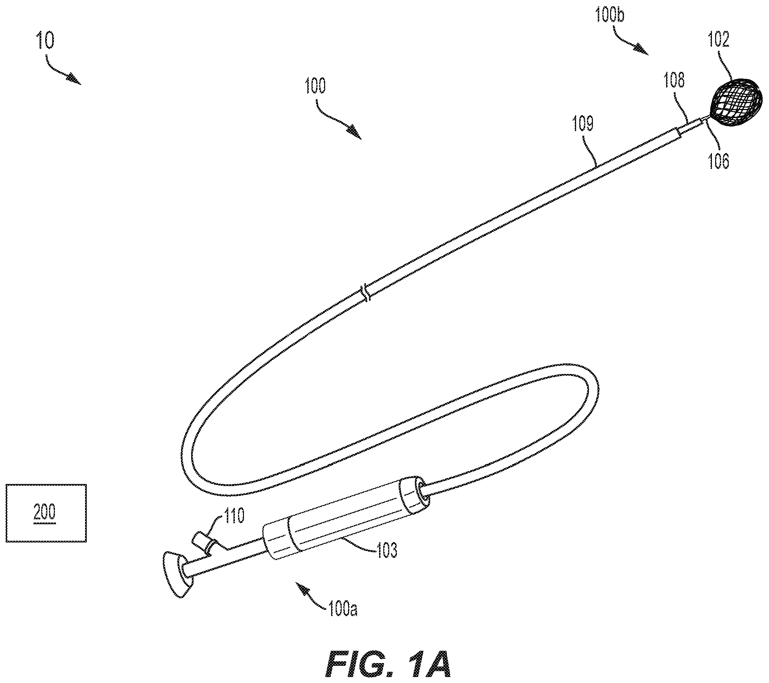

Specific details of systems, devices, and methods for treating intracranial aneurysms in accordance with embodiments of the present technology are described herein with reference to FIGS. 1A-15C. Although these systems, devices, and methods may be described herein primarily or entirely in the context of treating saccular intracranial aneurysms, other contexts are within the scope of the present technology. For example, suitable features of described systems, devices, and methods for treating saccular intracranial aneurysms can be implemented in the context of treating non-saccular intracranial aneurysms, abdominal aortic aneurysms, thoracic aortic aneurysms, renal artery aneurysms, arteriovenous malformations, tumors (e.g. via occlusion of vessel(s) feeding a tumor), perivascular leaks, varicose veins (e.g. via occlusion of one or more truncal veins such as the great saphenous vein), hemorrhoids, and sealing endoleaks adjacent to artificial heart valves, covered stents, and abdominal aortic aneurysm devices among other examples. Furthermore, it should be understood, in general, that other systems, devices, and methods in addition to those disclosed herein are within the scope of the present disclosure. For example, systems, devices, and methods in accordance with embodiments of the present technology can have different and/or additional configurations, components, procedures, etc. than those disclosed herein. Moreover, systems, devices, and methods in accordance with embodiments of the present disclosure can be without one or more of the configurations, components, procedures, etc. disclosed herein without deviating from the present technology.

### I. Overview of Systems of the Present Technology

FIG. 1A illustrates a view of a system 10 for treating intracranial aneurysms according to one or more embodiments of the present technology. As shown in FIG. 1A, the system 10 comprises a treatment system 100 and an embolic kit 200 for use with one or more components of the treatment system 100. The treatment system 100 may comprise an occlusive member 102 (shown in an expanded state) detachably coupled to a delivery system, and the delivery system may be configured to intravascularly position the occlusive member 102 within an aneurysm. The embolic kit 200 may comprise one or more substances or devices that alone or in combination form an embolic element that is configured to co-occupy the internal volume of the aneurysm with the occlusive member 102. In some embodiments, the treatment system 100 may be configured to deliver the embolic element (and/or one or more precursors thereof) to the aneurysm. Additionally or alternatively, the system 10 may include a separate delivery system (not shown) for delivering the embolic element (and/or one or more precursors thereof) to the aneurysm cavity.

As shown in FIG. 1A, the treatment system 100 has a proximal portion 100a configured to be extracorporeally positioned during treatment and a distal portion 100b configured to be intravascularly positioned within a blood vessel (such as an intracranial blood vessel) at a treatment site at or proximate an aneurysm. The treatment system 100 may include a handle 103 at the proximal portion 100a, the occlusive member 102 at the distal portion 100b, and a plurality of elongated shafts or members extending between the proximal and distal portions 100a and 100b. In some embodiments, such as that shown in FIG. 1A, the treatment system 100 may include a first elongated shaft 109 (such as a guide catheter or balloon guide catheter), a second elongated shaft 108 (such as a microcatheter) configured to be slidably disposed within a lumen of the first elongated shaft 109, and an elongated member 106 configured to be slidably disposed within a lumen of the second elongated shaft 108. In some embodiments, the treatment system 100 does not include the first elongated shaft 109 and only includes the second elongated shaft 108.

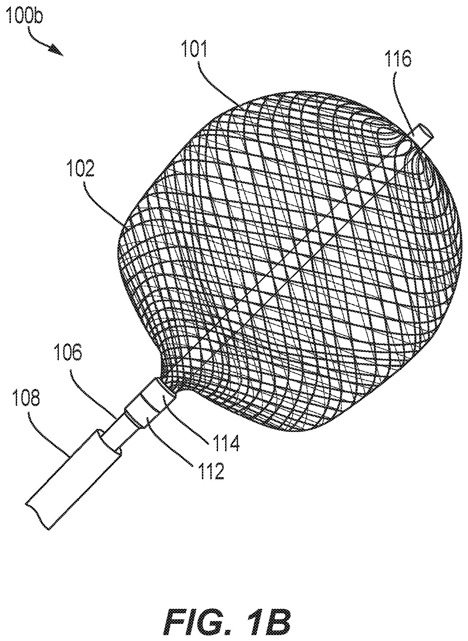

FIG. 1B is an enlarged view of the distal portion 100b of the treatment system 100. Referring to FIGS. 1A and 1B together, the occlusive member 102 may be detachably coupled to a distal end of the elongated member 106. For example, the elongated member 106 may include a first coupler 112 at its distal end, and the occlusive member 102 may include a second coupler 114 configured to detachably couple with the first coupler 112. The treatment system 100 may further comprise a conduit 116 extending from the handle 103 (for example, via port 110) distally to the distal portion 100b of the treatment system 100. The conduit 116 is configured to deliver the embolic element (and/or one or more precursors thereof) through one or more components of the delivery system (e.g., the first or second elongated shafts 109, 108, the elongated member 106, etc.) to a position at the exterior of the occlusive member 102. As such, the embolic element may be positioned between the occlusive member 102 and an inner wall of the aneurysm cavity, as described in greater detail below.

According to some embodiments, the second elongated shaft 108 is generally constructed to track over a conventional guidewire in the cervical anatomy and into the cerebral vessels associated with the brain and may also be chosen according to several standard designs that are generally available. Accordingly, the second elongated shaft 108 can have a length that is at least 125 cm long, and more particularly may be between about 125 cm and about 175 cm long. In some embodiments, the second elongated shaft 108 may have an inner diameter of about 0.0381 cm (0.015 inches), 0.043 cm (0.017 inches), about 0.053 cm (0.021 inches), or about 0.069 cm (0.027 inches). Other designs and dimensions are contemplated.

The elongated member 106 can be movable within the first and/or second elongated shafts 109, 108 to position the occlusive member 102 at a desired location. The elongated member 106 can be sufficiently flexible to allow manipulation, e.g., advancement and/or retraction, of the occlusive member 102 through tortuous passages. Tortuous passages can include, for example, catheter lumens, microcatheter lumens, blood vessels, urinary tracts, biliary tracts, and airways. The elongated member 106 can be formed of any material and in any dimensions suitable for the task(s) for which the system is to be employed. In some embodiments, the elongated member 106 can comprise a solid metal wire. In some embodiments, the elongated member 106 may comprise any other suitable form of shaft such as an elongated tubular shaft.

In some embodiments, the elongated member 106 can comprise stainless steel, nitinol, cobalt chrome, or other metal or alloy. In some embodiments, the elongated member 106 can be surrounded over some or all of its length by a coating, such as, for example, polytetrafluoroethylene. The elongated member 106 may have a diameter that is generally constant along its length, or the elongated member 106 may have a diameter that tapers radially inwardly, along at least a portion of its length, as it extends in a distal direction.

According to several embodiments, the conduit 116 may be a catheter or elongated shaft that is delivered separately from the second elongated shaft 108.

### A. Selected Examples of Occlusive Members

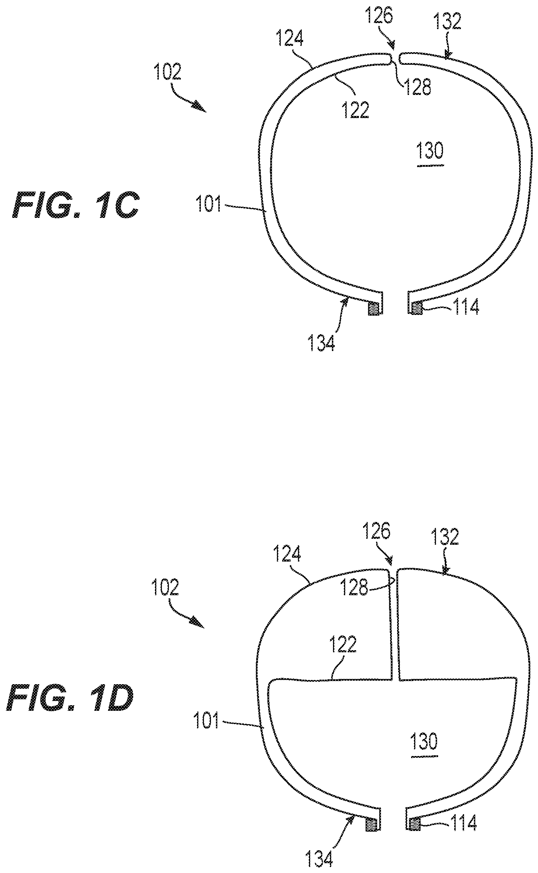

FIG. 1C is a sectioned view of the occlusive member 102, shown in an expanded state and detached from the treatment system 100. Referring to FIGS. 1B and 1C, the occlusive member 102 may comprise an expandable element having a low-profile or constrained state while positioned within a catheter (such as the second elongated shaft 108) for delivery to the aneurysm and an expanded state in which the expandable element is configured to be positioned within an aneurysm (such as a cerebral aneurysm).

According to some embodiments, the occlusive member 102 may comprise a mesh 101 formed of a plurality of braided filaments that have been heat-set to assume a predetermined shape enclosing an interior volume 130 when the mesh 101 is in an expanded, unconstrained state. Example shapes include a globular shape, such as a sphere, a prolate spheroid, an oblate spheroid, and others. As depicted in FIG. 1C, the mesh 101 may have inner and outer layers 122, 124 that have proximal ends fixed relative to one another at the second coupler 114 and meet distally at a distal fold 128 surrounding an aperture 126. While the inner and outer layers 122, 124 are depicted spaced apart from one another along their lengths, the inner and outer layers 122, 124 may be in contact with one another along all or a portion of their lengths. For example, the inner layer 122 may press radially outwardly against the outer layer 124. In some embodiments, the occlusive member 102 may be formed of a single layer or mesh or braid.

In some embodiments, the inner and outer layers 122, 124 have their distal ends fixed relative to one another at a distal coupler and meet proximally at a proximal fold surrounding an aperture. In any case, in some embodiments the conduit 116 may be configured to be slidably positioned through some or all of the second coupler 114, the interior volume 130 of the expanded mesh 101, and the opening 126.

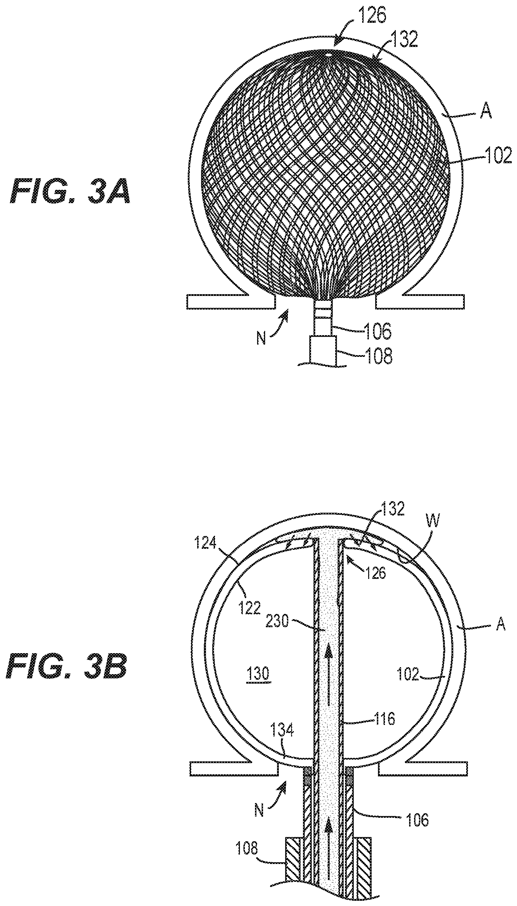

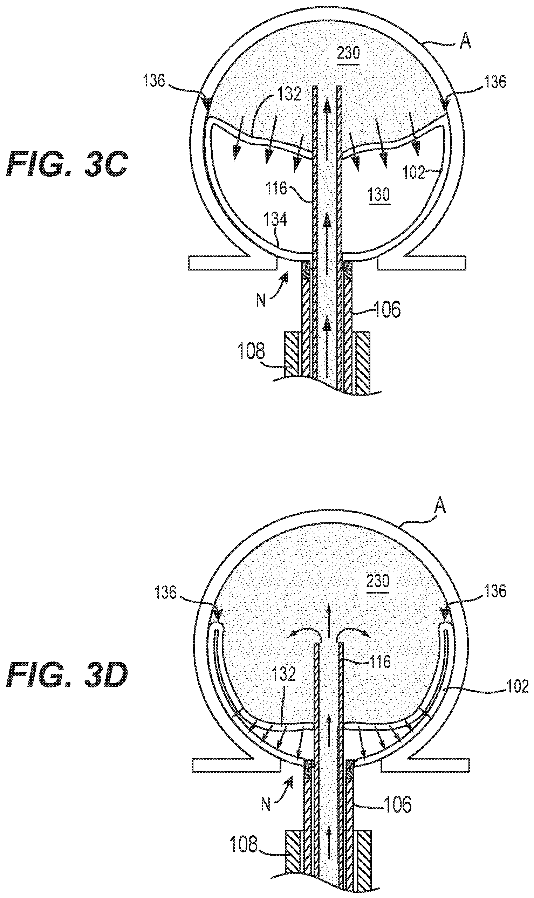

The inner and outer layers 122 and 124 may conform to one another at the distal portion (for example as shown in FIG. 1C) to form a curved distal surface. For example, at least at the distal portion of the occlusive member 102, the inner and outer layers 122 and 124 may extend distally and radially inwardly, towards the aperture 126. In some embodiments, the outer and/or inner layers 122 and 124 extend distally and radially outwardly from the second coupler 114, then extend distally and radially inwardly up to a distal terminus of the occlusive member 102 (e.g., the fold 128). The occlusive member 102 and/or layers thereof may be curved along its entire length, or may have one or more generally straight portions. In some embodiments, the curved surface transitions to a flat or substantially flat, distal-most surface that surrounds the aperture 126. In some embodiments, the curved surface transitions to a distal-most surface that surrounds the aperture 126 and has a radius of curvature that is greater than the average radius of curvature of the rest of the occlusive member 102. Having a flat or substantially flat distal surface, or a distal surface with a radius of curvature that is greater than the average radius of curvature of the rest of the occlusive member 102, may be beneficial for delivering the embolic element 230 in that it creates a small gap between the distal surface of the occlusive member 102 and the dome of the aneurysm A (see, for example, FIG. 3B). In some embodiments, the surface of the occlusive member 102 surrounding the aperture 126 is curved and/or has generally the same radius of curvature as the remainder of the occlusive member 102.

In any case, the inner layer 124 may have a shape that substantially conforms to the shape of the outer layer 124, or the inner and outer layers 122, 124 may have different shapes. For example, as shown in FIG. 1D, the inner layer 122 may have a diameter or cross-sectional dimension that is less than the outer layer 124. Such a configuration may be beneficial in that the embolic element 230 experiences less resistance, at least initially, when pushing the distal wall of the occlusion member 102 downwardly towards the neck (as described in greater detail below).

In any case, both the proximal portion and the distal portion of the mesh 101 can form generally closed surfaces. However, unlike at the proximal portion of the mesh 101, the portion of the filaments at or near the fold 128 at the distal portion of the mesh 101 can move relative to one another. As such, the distal portion of the mesh 101 has both the properties of a closed end and also some properties of an open end (like a traditional stent), such as some freedom of movement of the distal-most portions of the filaments and an opening through which the conduit 116, a guidewire, guidetube, or other elongated member may pass through.

In some embodiments, each of the plurality of filaments have a first end positioned at the proximal portion of the mesh 101 and a second end also positioned at the proximal portion of the mesh 101. Each of the filaments may extend from its corresponding first end distally along the body of the mesh 101 to the fold 128, invert, then extend proximally along the mesh body to its corresponding second end at the proximal portion of the mesh 101. As such, each of the plurality of filaments have a first length that forms the inner layer 122 of the mesh 101, a second length that forms the outer layer 124 of the mesh 101, and both first and second ends fixed at the proximal portion of the mesh 101. In some embodiments, the occlusive member 102 may comprise a mesh formed of a single layer, or a mesh formed of three or more layers.

In some embodiments, the distal end surface of the mesh 101 is completely closed (i.e., does not include an aperture). In some embodiments the filaments are fixed relative to the at both the proximal and distal ends of the occlusive member 102.

The mesh 101 may be formed of metal wires, polymer wires, or both, and the wires may have shape memory and/or superelastic properties. The mesh 101 may be formed of 24, 32, 36, 48, 64, 72, 96, 128, or 144 filaments. The mesh 101 may be formed of a range of filament or wire sizes, such as wires having a diameter of from about 0.0102 mm (0.0004 inches) to about 0.0508 mm (0.0020 inches), or of from about 0.0229 mm (0.0009 inches) to about 0.0305 mm (0.0012 inches). In some embodiments, each of the wires or filaments have a diameter of about 0.0102 mm (0.0004 inches), about 0.0127 mm (0.0005 inches), about 0.0152 mm (0.0006 inches), about 0.0178 mm (0.0007 inches), about 0.0203 mm (0.0008 inches), about 0.0229 mm (0.0009 inches), about 0.0254 mm (0.001 inches), about 0.0279 mm (0.0011 inches), about 0.0305 mm (0.0012 inches), about 0.0330 mm (0.0013 inches), about 0.0356 mm (0.0014 inches), about 0.0381 mm (0.0015 inches), about 0.0406 mm (0.0016 inches), about 0.0432 mm (0.0017 inches), about 0.0457 mm (0.0018 inches), about 0.0483 mm (0.0019 inches), or about 0.0508 mm (0.0020 inches). In some embodiments, all of the filaments of the braided mesh 101 may have the same diameter. For example, in some embodiments, all of the filaments have a diameter of about 0.025 mm (0.001 inches). In some embodiments, some of the filaments may have different cross-sectional diameters. For example, some of the filaments may have a slightly thicker diameter to impart additional strength to the braided layers. In some embodiments, some of the filaments can have a diameter of about 0.0254 mm (0.001 inches), and some of the filaments can have a diameter of greater than 0.0254 mm (0.001 inches). The thicker filaments may impart greater strength to the braid without significantly increasing the device delivery profile, with the thinner wires offering some strength while filling-out the braid matrix density.

The occlusive member 102 can have different shapes and sizes in an expanded, unconstrained state. For example, the occlusive member 102 may have a bullet shape, a barrel-shape, an egg shape, a dreidel shape, a bowl shape, a disc shape, a cylindrical or substantially cylindrical shape, a barrel shape, a chalice shape, etc.

### B. Selected Examples of Embolic Kits

The embolic kit 200 may include one or more precursors for creation of a liquid embolic. For example, the embolic kit 200 may include a first container 202 containing a first precursor material 203 (shown schematically), a second container 204 containing a second precursor material 205 (also shown schematically), and a mixing device 206 suitable for mixing the first and second precursor materials 203, 205. The mixing device 206 can include mixing syringes 208 (individually identified as mixing syringes 208a, 208b) and a coupler 210 extending between respective exit ports (not shown) of the mixing syringes 208. The mixing syringes 208a, 208b each include a plunger 212 and a barrel 214 in which the plunger 212 is slidably received.

The embolic kit 200 can further include an injection syringe 216 configured to receive a mixture of the first and second precursor materials 203, 205 and deliver the mixture to a proximal portion 100b of the treatment assembly 100. The injection syringe 216 can include a barrel 220, an exit port 222 at one end of the barrel 220, and a plunger 224 slidably received within the barrel 220 via an opposite end of the barrel 220. The handle 103 of the treatment system 100 may have a coupler configured to form a secure fluidic connection between the lumen and the exit port 222 of the injection syringe 216.

The first and second precursor materials 203, 205 can include a biopolymer and a chemical crosslinking agent, respectively. The chemical crosslinking agent can be selected to form covalent crosslinks between chains of the biopolymer. In some embodiments, the biopolymer of the first precursor material 203 includes chitosan or a derivative or analog thereof, and the chemical crosslinking agent of the second precursor material 205 includes genipin or a derivative or analog thereof. Other suitable crosslinking agents for use with chitosan include glutaraldehyde, functionalized polyethylene glycol, and derivatives and analogs thereof. In other embodiments, the biopolymer of the first precursor material 203 can include collagen or a derivative or analog thereof, and the chemical crosslinking agent of the second precursor material 205 can include hexamethylene diisocyanate or a derivative or analog thereof. Alternatively or in addition, genipin or a derivative or analog thereof can be used as a chemical crosslinking agent for a collagen-based biopolymer. In still other embodiments, the biopolymer of the first precursor material 203 and the chemical crosslinking agent of the second precursor material 205 can include other suitable compounds alone or in combination.

Mixing the biopolymer of the first precursor material 203 and the chemical crosslinking agent of the second precursor material 205 can initiate chemical crosslinking of the biopolymer. After the first and second precursor materials 203, 205 are mixed, chemical crosslinking of the biopolymer occurs for enough time to allow the resulting embolic element 230 be delivered to the aneurysm before becoming too viscous to move through the lumen of the conduit 116. In addition, the period of time during which chemical crosslinking of the biopolymer occurs can be short enough to reach a target deployed viscosity within a reasonable time (e.g., in the range of 10-60 minutes; or at most 40 minutes, 30 minutes, 20 minutes, or 10 minutes) after delivery. The target deployed viscosity can be high enough to cause an agglomeration of the embolic element 230 to remain within the internal volume of the aneurysm without reinforcing the neck.

In at least some cases, the biopolymer has a non-zero degree of chemical crosslinking within the first precursor material 203 before mixing with the chemical crosslinking agent. This can be useful, for example, to customize the curing window for the embolic element 230 so that it corresponds well with an expected amount of time needed to deliver the material to the aneurysm. The degree of chemical crosslinking of the biopolymer within the first precursor material 203 before mixing with the chemical crosslinking agent, the ratio of the biopolymer to the chemical crosslinking agent, and/or one or more other variables can be selected to cause the embolic element 230 to have a viscosity suitable for delivery to the aneurysm via the lumen of the conduit 116 for a suitable period of time (e.g., a period within a range from 10 minutes to 40 minutes) after mixing of the first and second precursor materials 203, 205. In at least some cases, the first and second precursor materials 203, 205 are mixed in proportions that cause a weight ratio of the biopolymer to the chemical crosslinking agent in the resulting embolic element 230 to be within a range from 10:1 to 100:1, such as from 10:1 to 30:1, or from 15:1 to 50:1, or from 15:1 to 25:1. In a particular example, the first and second precursor materials 203, 205 are mixed in proportions that cause a weight ratio of the biopolymer to the chemical crosslinking agent in the resulting embolic element 230 to be 30:1.

Use of a biopolymer instead of an artificial polymer in the first precursor material 203 may be advantageous because biopolymers tend to be more readily bioabsorbed than artificial polymers and/or for other reasons. Furthermore, use of a chemical crosslinking agent instead of a physical crosslinking agent (i.e., a crosslinking agent that forms noncovalent crosslinks between chains of the biopolymer) in the second precursor material 205 may be advantageous because chemically crosslinked polymers tend to be more cohesive than physically crosslinked polymers and/or for other reasons. In the context of forming a tissue scaffold within an aneurysm, high cohesiveness of the embolic element 230 may be more important than it is in other contexts to secure the cured embolic element 230 within the aneurysm 302. For example, high cohesiveness of the embolic element 230 may reduce or eliminate the possibility of a piece of the embolic element 230 breaking free and entering a patient's intracerebral blood stream during delivery.

The first and second precursor materials 203, 205 may include other components and/or the system 200 may include other precursor materials intended for mixing with the first and second precursor materials 203, 205. For example, the first, second, and/or another precursor material may include a physical crosslinking agent. The presence of a physical crosslinking agent may be useful to form physical crosslinks that complement chemical crosslinks from the chemical crosslinking agent. The combination of chemical and physical crosslinks may enhance the cohesiveness of the embolic element 230. Suitable physical crosslinking agents for use with chitosan-based biopolymers include β glycerophosphate, mannitol, glucose, and derivatives and analogs thereof. In these and other cases, the embolic element 230 may include multiple chemical crosslinking agents and/or multiple physical crosslinking agents.

A contrast agent is another component that may be added to the precursor materials. The presence of a contrast agent within the embolic element 230 can be useful to visualize delivery of the embolic element 230 using fluoroscopy. One problem with using conventional platinum coils in intracranial aneurysms is that the persistent radiopacity of the coils tends to interfere with visualizing other aspects of the treatment in follow-up imaging. For example, the presence of platinum coils within an aneurysm may make it difficult or impossible to detect by fluoroscopy the presence of blood-carried contrast agent that would otherwise indicate recanalization. In at least some embodiments of the present technology, a contrast agent within the embolic element 230 is selected to provide radiopacity that diminishes over time. For example, the contrast agent may initially be radiopaque to facilitate delivery of the embolic element 230 and then become less radiopaque to facilitate follow-up imaging. In a particular example, the first, second, and/or another precursor material includes iohexol or a derivative or analog thereof as a suitable contrast agent.

In animal studies, the liquid embolics of the present technology were shown to provide (a) complete or nearly complete volumetric filling of the aneurysm internal volume, and (b) complete or nearly complete coverage of the aneurysm neck with new endothelial tissue. These features, among others, are expected to result in a lower recanalization rate than that of platinum coil treatments and faster aneurysm occlusion than that of flow diverters. Furthermore, the injectable scaffold material is expected to be bioabsorbed and thereby reduced in volume over time. Thus, unlike platinum coils, the injectable scaffold is expected to have little or no long-term mass effect. Furthermore, the injectable scaffold material can be configured to have diminishing radiopacity; therefore, when so configured it will not interfere future CT and MRI imaging and procedures. Embodiments of the present technology can have these and/or other features and advantages relative to conventional counterparts whether or not such features and advantages are described herein.

In some embodiments, the embolic kit 200 and/or embolic element 230 may be any embolic or occlusive device, such as one or more embolic coils, polymer hydrogel(s), polymer fibers, mesh devices, or combinations thereof. The embolic kit 200 may include one or more precursors that, once mixed together, form the embolic element 230 that remains within the aneurysm. In some embodiments, the embolic kit 200 may include the embolic element pre-mixed.

### II. Selected Methods for Treating Aneurysms

FIGS. 3A-3G depict an example method for treating an aneurysm A with the systems 10 of the present technology. To begin, a physician may intravascularly advance the second elongated shaft 108 towards an intracranial aneurysm (or other treatment location such as any of those described herein) with the occlusive member 102 in a low-profile state. A distal portion of the second elongated shaft 108 may be advanced through a neck N of the aneurysm A to locate a distal opening of the second elongated shaft 108 within an interior cavity of the aneurysm A. The elongated member 106 may be advanced distally relative to the second elongated shaft 108 to push the occlusive member 102 through the opening at the distal end of the second elongated shaft 108, thereby releasing the occlusive member 102 from the shaft 108 and allowing the occlusive member 102 to self-expand into a first expanded state.

FIG. 3A shows the occlusive member 102 in a first expanded state, positioned in an aneurysm cavity and still coupled to the elongated member 106. As shown in FIG. 3A, in the first expanded state, the occlusive member 102 may assume a predetermined shape that encloses an internal volume 130 (see FIG. 1C). In this first expanded state, the occlusive member 102 may generally conform to the shape of the aneurysm A. As illustrated in FIG. 3B with the occlusive member 102 and delivery system shown in cross-section, the conduit 116 may be advanced through the internal volume 130 of the occlusive member 102 such that a distal opening of the conduit 116 is at or distal to the aperture 126 at the distal portion of the occlusive member 102. The embolic element 230 may be delivered through the conduit 116 to a space between the occlusive member 102 and an inner surface of the aneurysm wall W.

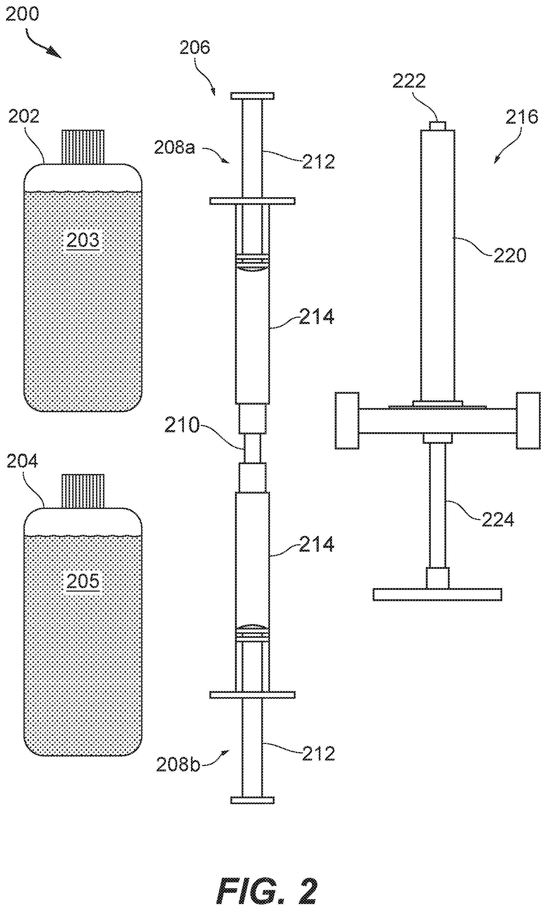

In some embodiments, the method includes mixing the first and second precursor materials 203, 205 (FIG. 2) to form the embolic element 230. Mixing of the first and second precursor materials 203, 205 may occur prior to introducing the embolic element 230 to the treatment system 100 and/or during delivery of the embolic element through the conduit 116 to the aneurysm. In a particular example, the first precursor material 203 is loaded into one of the barrels 214, the second precursor materials 205 is loaded into the other barrel 214, and the mixing syringes 208 are coupled via the coupler 210. To mix the first and second precursor materials 203, 205, the plungers 212 are alternately depressed, thereby causing the first and second precursor materials 203, 205 to move repeatedly from one barrel 214 to the other barrel 214. After suitably mixing the precursor materials, the resulting embolic element 230 can be loaded into the barrel 220 of the injection syringe 216. The injection syringe 216 may then be coupled to a proximal end of the conduit 116 to deliver the embolic element 230 through the conduit 116 and into the aneurysm A. As the embolic element 230 passes through the lumen of the conduit 116, chemical crosslinking of the biopolymer can continue to occur.

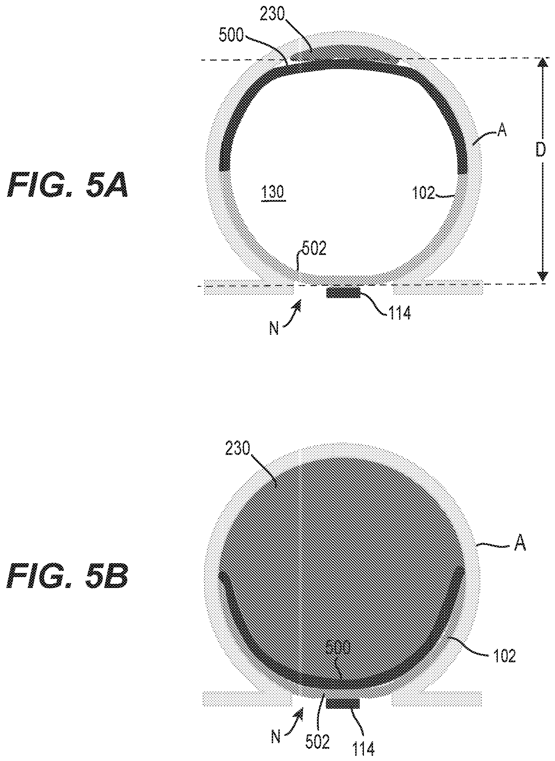

Still with reference to FIG. 3B, as the embolic element 230 is delivered between the dome of the aneurysm A and the distal portion 132 of the wall of the occlusive member 102, pressure builds between the aneurysm wall W and the occlusive member 102. As shown in the progression of FIGS. 3B-3D, when the forces on the occlusive member 102 reach a threshold level, the embolic element 230 pushes the distal wall 132 downwardly towards the neck N of the aneurysm A. The embolic element 230 exerts a substantially uniform pressure across the distal surface of the occlusive member 102 that collapses the occlusive member 102 inwardly on itself such that the rounded distal wall 132 transitions from concave towards the neck N of the aneurysm A to convex towards the neck N. The pressure and inversion of the distal portion of the wall 132 creates an annular fold 136 that defines the distal-most edge of the occlusive member 102. As the occlusive member 102 continues to invert, the position of the fold 136 moves towards the neck N, which continues until a distal-most half of the occlusive member 102 has inverted. In some embodiments, the occlusive member 102 may include one or more portions configured to preferentially flex or bend such that the occlusive member 102 folds at a desired longitude (for example, as discussed in greater detail below with respect to FIGS. 5-7J. Moreover, as the occlusive member 102 collapses, a distance between the wall at the distal portion 132 and the wall at the proximal portion decreases, and thus the internal volume 130 of the occlusive member 102 also decreases. As the occlusive member 102 collapses, the conduit 116 may be held stationary, advanced distally, and/or retracted proximally.

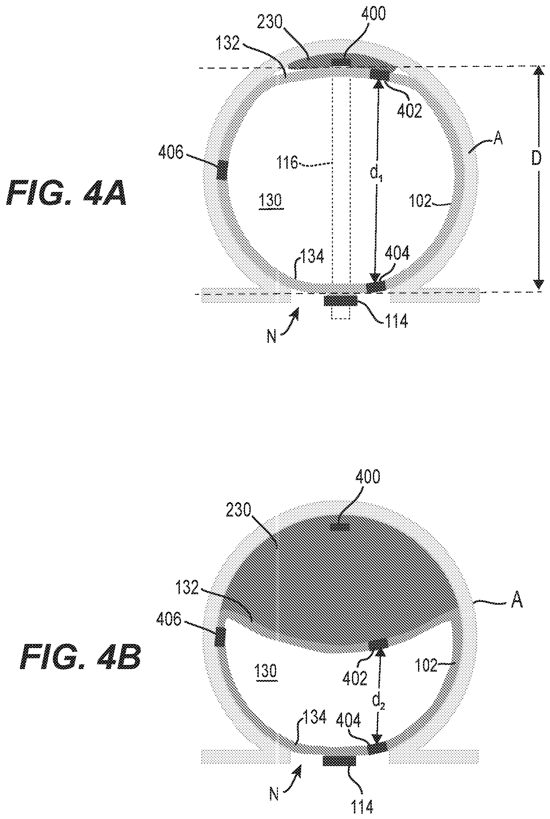

During and after delivery of the embolic element 230, none or substantially none of the embolic element 230 migrates through the pores of the occlusive member 102 and into the internal volume 130. Said another way, all or substantially all of the embolic element 230 remains at the exterior surface or outside of the occlusive member 102. Compression of the occlusive member with the embolic element 230 provides a real-time "leveling" or "aneurysm-filling indicator" to the physician under single plane imaging methods (such as fluoroscopy) so that the physician can confirm at what point the volume of the aneurysm is completely filled. Additional details regarding devices, systems, and methods for monitoring and/or confirming deployment are described below with reference to FIGS. 4A-5B. It is beneficial to fill as much space in the aneurysm as possible, as leaving voids within the aneurysm sac may cause delayed healing and increased risk of aneurysm recanalization and/or rupture. While the scaffolding provided by the occlusive member 102 across the neck helps thrombosis of blood in any gaps and healing at the neck, the substantial filling of the cavity prevents rupture acutely and does not rely on the neck scaffold (i.e., the occlusive member 102). Confirmation of complete or substantially complete aneurysm filling under single plane imaging cannot be provided by conventional devices.

Once delivery of the embolic element 230 is complete, the conduit 116 may be withdrawn. In some embodiments, the embolic element 230 may fill greater than 40% of the aneurysm sac volume. In some embodiments, the embolic element 230 may fill greater than 50% of the aneurysm sac volume. In some embodiments, the embolic element 230 may fill greater than 60% of the aneurysm sac volume. In some embodiments, the embolic element may fill greater than 65%, 70%, 75%, 80%, 85%, or 90% of the aneurysm sac volume.

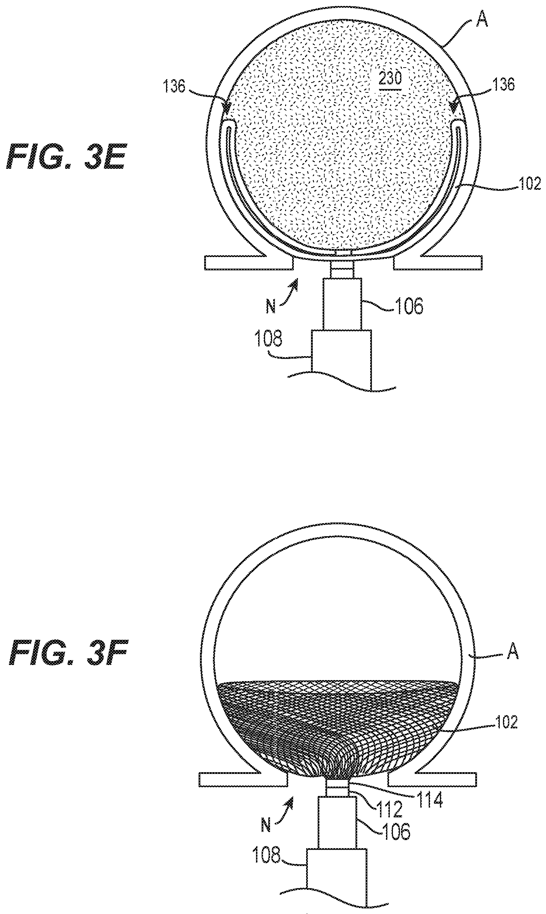

FIG. 3E shows a second expanded state of the occlusive member 102, shown in cross-section, with the embolic element 230 occupying the remaining volume of the aneurysm A. FIG. 3F shows the occlusive member 102 in full with the embolic element 230 removed so the second shape of the occlusive member 102 is visible. As shown, the embolic element 230 may be delivered until the occlusive member 102 is fully-collapsed such that the occlusive member 102 has substantially no internal volume.

In the second expanded state, the occlusive member 102 may form a bowl shape that extends across the neck of the aneurysm A. The wall of the occlusive member 102 at the distal portion may now be positioned in contact with or immediately adjacent the wall of the occlusive member 102 at the proximal portion. The distal wall 132 may be in contact with the proximal wall 134 along all or substantially all of its length. In some embodiments, the distal wall 132 may be in contact with the proximal wall 134 along only a portion of its length, while the remainder of the length of the distal wall 132 is in close proximity-but not in contact with-the proximal wall 134.

Collapse of the occlusive member 102 onto itself, towards the neck N of the aneurysm, may be especially beneficial as it doubles the number of layers across the neck and thus increases occlusion at the neck N. For example, the distal wall 132 collapsing or inverting onto the proximal wall 134 may decrease the porosity of the occlusive member 102 at the neck N. In those embodiments where the occlusive member 102 is a mesh or braided device such that the distal wall 132 has a first porosity and the proximal wall 134 has a second porosity, deformation of the distal wall 132 onto or into close proximity within the proximal wall 134 decreases the effective porosity of the occlusive member 102 over the neck N. The resulting multi-layer structure thus has a lower porosity than the individual first and second porosities. Moreover, the embolic element 230 along the distal wall 132 provides additional occlusion. In some embodiments, the embolic element 230 completely or substantially completely occludes the pores of the adjacent layer or wall of the occlusion member 102 such that blood cannot flow past the embolic element 230 into the aneurysm cavity. It is desirable to occlude as much of the aneurysm as possible, as leaving voids of gaps can allow blood to flow in and/or pool, which may continue to stretch out the walls of aneurysm A. Dilation of the aneurysm A can lead to recanalization and/or herniation of the occlusive member 102 and/or embolic element 230 into the parent vessel and/or may cause the aneurysm A to rupture. Both conditions can be fatal to the patient.

In those embodiments where the wall of the occlusive member 102 comprises an inner and outer layer, the deformed or second shape of the occlusive member 102 forms four layers over the neck N of the aneurysm A In those embodiments where the wall of the occlusive member 102 comprises a single layer, the deformed or second shape of the occlusive member 102 forms two layers over the neck N of the aneurysm A As previously mentioned, the neck coverage provided by the doubled layers provides additional surface area for endothelial cell growth, decreases the porosity of the occlusive member 102 at the neck N (as compared to two layers or one layer), and prevents herniation of the embolic element 230 into the parent vessel. During and after delivery, the embolic element 230 exerts a substantially uniform pressure on the occlusive member 102 towards the neck N of the aneurysm A, thereby pressing the portions of the occlusive member 102 positioned adjacent the neck against the inner surface of the aneurysm wall such that the occlusive member 102 forms a complete and stable seal at the neck N.

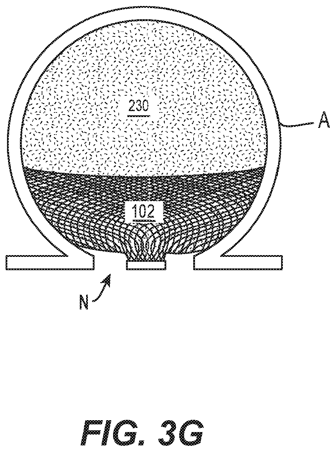

As shown in FIG. 3G, the first coupler 112 may be detached from the second coupler 114 and the elongated member 106 and second elongated shaft 108 may be withdrawn, thereby leaving the occlusive member 102 and embolic element 230 implanted within the aneurysm A.

Over time natural vascular remodeling mechanisms and/or bioabsorption of the embolic element 230 may lead to formation of a thrombus and/or conversion of entrapped thrombus to fibrous tissue within the internal volume of the aneurysm A. These mechanisms also may lead to cell death at a wall of the aneurysm and growth of new endothelial cells between and over the filaments or struts of the occlusive device 102. Eventually, the thrombus and the cells at the wall of the aneurysm may fully degrade, leaving behind a successfully remodeled region of the blood vessel.

In some embodiments, contrast agent can be delivered during advancement of the occlusive member 102 and/or embolic element 230 in the vasculature, deployment of the occlusive member 102 and/or embolic element 230 at the aneurysm A, and/or after deployment of the occlusive member 102 and/or embolic element 230 prior to initiation of withdrawal of the delivery system. The contrast agent can be delivered through the second elongated shaft 108, the conduit 116, or through another catheter or device commonly used to delivery contrast agent. The aneurysm (and devices therein) may be imaged before, during, and/or after injection of the contrast agent, and the images may be compared to confirm a degree of occlusion of the aneurysm.

According to some aspects of the technology, the system 10 may comprise separate first and second elongated shafts (e.g., microcatheters) (not shown), the first dedicated to delivery of the embolic element, and the second dedicated to the delivery of the occlusive member. In example methods of treating an aneurysm, the first elongated shaft may be intravascularly advanced to the aneurysm and through the neck such that that a distal tip of the first elongated shaft is positioned within the aneurysm cavity. In some embodiments, the first elongated shaft may be positioned within the aneurysm cavity such that the distal tip of the shaft is near the dome of the aneurysm.

The second elongated shaft containing the occlusive member (such as occlusive member 102) may be intravascularly advanced to the aneurysm and positioned within the aneurysm cavity adjacent the first elongated shaft. The occlusive member may then be deployed within the aneurysm sac. As the occlusive member is deployed, it pushes the first elongated shaft outwardly towards the side of the aneurysm, and when fully deployed the occlusive member holds or "jails" the first elongated shaft between an outer surface of the occlusive member and the inner surface of the aneurysm wall.

The embolic element (such as embolic element 230) may then be delivered through the first elongated shaft to a position between the inner surface of the aneurysm wall and the outer surface of the occlusive member. For this reason, it may be beneficial to initially position the distal tip of the first elongated shaft near the dome (or more distal surface) of the aneurysm wall. This way, the "jailed" first elongated shaft will be secured by the occlusive member such that the embolic element gradually fills the open space in the aneurysm sac between the dome and the occlusive member. As described elsewhere herein, the filling of the embolic element pushes and compresses the occlusive member against the tissue surrounding the aneurysm neck as the space in the sac above the occlusive member is being filled from the dome to the neck. Also as described elsewhere herein, the compression of the occlusive member with the embolic element provides a "leveling or aneurysm filling indicator" which is not provided by conventional single plane imaging methods. The filling of the embolic element may complete, for example, when it occupies about 50-80% of the volume of the aneurysm.

### III. Selected Devices, Systems, and Methods for Monitoring Deployment

Proper deployment of the embolic element 230 and the occlusive member 102 can be monitored and/or confirmed using one or more medical imaging techniques, such as fluoroscopy. FIGS. 4A-5B illustrate examples of various types of fluoroscopic images that may be employed by a physician at different stages of deployment to monitor the position of the occlusive member 102 within the aneurysm A, monitor the degree of filling of the aneurysm A with the embolic element 230, and/or confirm a degree of occlusion of the aneurysm A by the deployed system. As described in greater detail below, the devices and systems of the present technology may be configured to provide unique visual indicators that provide confirmation to the physician via one or more medical imaging techniques regarding a degree of occlusion of the aneurysm. As described in greater detail below, the visual indicators may include a particular change in shape of all or a portion of the occlusive member 102, a particular change in relative position of one or more radiopaque markers on the occlusive member 102 and/or delivery system (such as the conduit 116), a particular change in shape of the embolic element 230, and others.

Although the following discussion is made with reference to the two-dimensional images shown in FIGS. 4A-5B, the systems and methods of the present technology can be employed with three-dimensional imaging techniques. Moreover, FIGS. 4A-5B represent a two-dimensional image in which only a slice of the aneurysm (and devices therein) is visible. While in some cases the inner and outer layers of the occlusive member 102 (when such are present) may be distinguishable from one another in the radiographic image, in the present example the layers appear as one thick layer. As used herein, "proper deployment" or "successful deployment" may refer to (a) complete (e.g., greater than 80%) or substantially complete (e.g., greater than 50%) filling of the aneurysm A with the embolic element 230, (b) complete or substantially complete inversion or collapse of the occlusive member 102 onto itself over the neck N of the aneurysm A, (c) or both.

The occlusive member 102 may include one or more radiopaque markers, such as markers 402, 404, 406, and 114 (referred to collectively as "markers 401") shown in FIGS. 4A-4C. The markers 401 may be disposed about the occlusive member 102 in a specific spatial arrangement such that relative movement of the markers is indicative of a degree of stage of deployment of the occlusive member 102 and/or embolic element 230. The markers 401 may be positioned at any location along the occlusive member 102. For example, the occlusive member 102 may include one or more radiopaque markers 402 at or along its distal wall 132 (only one shown for ease of illustration), one or more radiopaque markers 404 at or along its proximal wall 134 (only one shown for ease of illustration), and one or more radiopaque markers 406 at or along the intermediate portion of the wall (only one shown for ease of illustration). Moreover, the coupler 114 of the occlusive member 102 may be radiopaque. The markers 401 may be positioned at one, some, or all of the layers of the occlusive member 102 (at least in those embodiments where the occlusive member 102 includes multiple layers). In some embodiments, the individual markers 401 may comprise a radiopaque band or clip coupled to the one or more struts, filaments, wires, etc. of the occlusive member 102. In some embodiments, the individual markers 401 may comprise a radiopaque material coated on or otherwise incorporated into the wall of the occlusive member 102. The individual markers 401 may have the same or different shapes, lengths, and/or profiles.

In some embodiments, in addition to or instead of having one or more markers 401, the occlusive member 102 itself may be partially or completely formed of a radiopaque material, such as one or more radiopaque wires. In the example depicted in FIGS. 4A-4C, the occlusive member 102 is formed of a radiopaque material and also includes radiopaque markers 402, 404, 406. The occlusive member 102 is formed of a plurality of drawn-filled tube ("DFT") wires, which comprise a core formed of a radiopaque material (such as platinum) surrounded by an outer non-radiopaque material (at least relative to the core material). The markers 402, 404, 406 are completely formed of a radiopaque material and thus have a higher density of radiopaque material. As such, the markers 402, 404, 406 appear darker than the occlusive member 102 in the images. In some embodiments, the occlusive member 102 may have a radiopacity that is different than the radiopacity of one or more of the markers 402, 404, 406 such that the wall of the occlusive member 102 wall and the marker(s) 406 can be distinguished from one another on the radiographic image. The wall of the occlusive member 102 may be more or less radiopaque than one or more of the markers 402, 404, 406.

In some embodiments, one or more components of the delivery system may include one or more radiopaque markers. For example, the conduit 116 may include one or more radiopaque markers positioned along its length. In the embodiment depicted in FIGS. 4A-4C, the conduit 116 may include a radiopaque marker 400 positioned at or near its distal end. The conduit 116 may have one or more additional markers (not shown) positioned along its length, such as along the length of the conduit 116 that extends through the interior volume 130 of the occlusive member 102.

As shown in FIG. 4A, when the occlusive member 102 is first deployed (e.g., allowed to self-expand) within the aneurysm, the radiopaque marker(s) 402, 404, 406 of the occlusive member 102 will be in a first position relative to one another, and to the radiopaque marker(s) of the conduit 116. By way of example, markers 402 and 404 are separated by a first distance d₁ when the occlusive member 102 is first deployed. As the embolic element 230 is conveyed through the conduit 116 and into the aneurysm sac, the occlusive member 102 may deform as described previously with respect to FIGS. 3A-3G. This deformation can cause the radiopaque marker(s) 401 carried by the occlusive member 102 to move to a second position relative to one another. For example, the physician may confirm progression of deployment by observing that markers 402 and 404 are now separated by a distance d₂. The radiopaque marker(s) 401 may also move relative to the radiopaque marker(s) 400 of the conduit 116, which may remain in the same or substantially the same place within the aneurysm. By comparing an image of the radiopaque markers 400 and/or 401 in the first relative positions and an image of the radiopaque markers 400 and/or 401 in the second relative positions, a clinician can visually confirm that the embolic element 230 has filled a certain percentage of the aneurysm A.

For example, according to some aspects of the technology, confirmation of sufficient filling of the aneurysm (i.e., 50% or greater) may be indicated by one or more distal wall markers 402 moving into close proximity to one or more proximal wall markers 404 and/or touching one or more proximal wall markers 404. Because the embolic element 230 applies a generally uniform pressure across the distal wall 132 and pushes downwardly towards the neck N as it fills in the space between the occlusive member 102 and the aneurysm wall, the movement of one or more distal wall markers 402 to a position adjacent a proximal wall marker 404 indicates to a physician that the aneurysm A is substantially filled (e.g., 50% or greater) with the embolic element 230. This relative positioning also indicates that the distal wall 132 is now providing additional occlusion at the neck N of the aneurysm and that the occlusive member 102 is in its second expanded shape. In some embodiments, the coupler 114 may be used as the proximal indicator instead of or in addition to the one or more proximal markers 404.

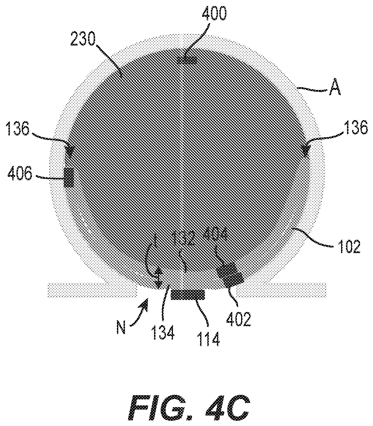

In some embodiments, confirmation of sufficient filling of the aneurysm (i.e., 50% or greater) may be indicated by one or more distal wall markers 402 moving away from the conduit marker 400 (or marker affixed to another component of the delivery system) by a predetermined distance. For example, when the occlusive member 102 is in the first expanded state or shape (FIG. 4A) the distal wall marker 402 may be adjacent the conduit marker 400. In the second expanded state or shape (FIG. 4C), the distal wall marker 402 may be separated from the conduit marker 400 by a distance that is generally equivalent to a diameter D of the occlusive member 102 in its expanded state while initially positioned in the aneurysm A. As explained above, such relative positioning of one or more distal wall markers 402 and conduit marker 400 indicates to a physician that the aneurysm A is substantially filled (e.g., 50% or greater) with the embolic element 230. This relative positioning also indicates that the distal wall 132 is now providing additional occlusion at the neck N of the aneurysm and that the occlusive member 102 is in its second expanded shape.

In some embodiments, one or more intermediate markers 406 may be used to confirm and/or monitor deployment. For example, one or more intermediate markers 406 may be positioned at or near a desired inversion plane of the occlusive member 102. In the present example using a generally spherical occlusive member 102 that deforms to assume a bowl shape, the inversion plane is at or near a midline of the occlusive member 102 in its expanded state. This is because, in a fully inverted state, the distal half of the occlusive member 102 will lie within/conform to the proximal half of the occlusive member 102 (as shown in FIG. 4C). As such, the midline of the occlusive member 102 is the desired plane of inversion. The occlusive member 102 may be radiopaque (as shown in FIGS. 4A-4C), but to a lesser extent than the intermediate marker(s) 406 such that the occlusive member 102 wall and the marker(s) 406 can be distinguished from one another on the radiographic image. As such, an image showing the top edge 136 (FIG. 4C) of the occlusive member 102 adjacent or at the intermediate marker(s) 406 may indicate that the aneurysm A is substantially filled (e.g., 50% or greater) with the embolic element 230. This relative positioning also indicates that the distal wall 132 is now providing additional occlusion at the neck N of the aneurysm and that the occlusive member 102 is in its second expanded shape.

The change in shape of the occlusive member 102 and/or change in position of different portions of the occlusive member 102 relatively to one another may also indicate proper deployment. As previously discussed, the occlusive member 102 assumes a first expanded shape when initially deployed and has a second expanded shape after deformation by the embolic element 230. In several embodiments, the second expanded shape represents a partially or completely inverted from of the first expanded shape, which can be confirmed on the radiographic image by observing the changing outline of the occlusive member 102. For instance, in the present example where the occlusive member 102 has a first expanded shape that is generally spherical, an image showing a C-shape (as shown in FIG. 4C) may indicate that the desired filling and/or deployment is complete. In a three-dimensional image, the second expanded shape may have a bowl shape. In some embodiments, confirmation of complete or substantially complete deployment may be indicated by the distal wall 500 being within a predetermined distance of the proximal wall 502.

In some embodiments, proper deployment may be confirmed by observing a distance between the inverted wall (here, distal wall 132) and the relatively stationary wall (here, proximal wall 134). As shown in FIG. 4C, when the distal wall 132 collapses down onto or near the proximal wall 134, the occlusive member 102 presents on the image as having twice the thickness at the proximal portion. Moreover, as the occlusive member 102 inverts, the density of the radiopaque material doubles, and thus the doubled-over portions of the occlusive member 102 appear darker on the image.

As shown in FIGS. 5A and 5B, in some embodiments, certain portions of the occlusive member 102 may be coated with a radiopaque material such that change in shape or orientation of those portions indicates a desired position of the occlusive member 102. For example, as shown in FIG. 5A, a distal-most half 500 of the occlusive member 102 may be coated with a radiopaque material while a proximal-most half 502 may not be coated or may otherwise be less radiopaque than the distal half 500. As such, confirmation of complete or substantially complete deployment may be indicated by the more radiopaque distal wall 500 being adjacent the proximal wall 502. For example, confirmation of complete or substantially complete deployment may be indicated by the distal wall 500 being within a predetermined distance of the proximal wall 502. Confirmation may also be gleaned from the distal wall 500 changing in shape from flat or convex (towards the dome) of the aneurysm A to concave.

A shape of the embolic element 230 may also provide an indication of deployment progress. For example, the shape of the lower (closer to the neck N) perimeter of the aneurysm A can be indicative of a degree of filling of the aneurysm with the embolic element 230 and/or degree of deformation of the occlusive member 102. As most aneurysms have a generally spherical or globular shape, a lower boundary of the embolic element 230 may have a decreasing radius of curvature as more is injected and more of the occlusive member 102 inverts. For example, in FIG. 4B, when the aneurysm A is partially filled with the embolic element 230 and the occlusive member 102 is only partially collapsed or inverted, the distal wall 132 has a first radius of curvature. In FIG. 4C, when the aneurysm A is substantially completely or completely filled, the distal wall 132 has a radius of curvature less than the radius of curvature of the distal wall 132 in the partially deformed state.

Additionally or alternatively, the degree of deployment of the occlusive member 102 and/or degree of filling of the aneurysm A can be further determined by injecting contrast into the parent blood vessel and imaging the aneurysm to determine how much of the contrast enters the aneurysm cavity. The shape of the
The devices, systems, and methods of the present technology may be particularly beneficial over conventional devices for two-dimensional imaging. In two dimensional imaging (such as fluoroscopy), the image may reflect only a slice or elevational view of the aneurysm (and device or substance therein). As such, any voids or gaps in filling may not be apparent in the slice because the image slice does not transect the void within the aneurysm A, or the cross-section or elevational view of the stagnated area may take on different shapes depending on how the image is observed. A physician may have to take a plurality of images to determine a general amount of filling in the aneurysm. In contrast, the occlusion members 102 of the present technology have a unique shape that dynamically adjusts to the introduction of an embolic element 230 in a predictable, measurable way that indicates a degree of filling of the embolic element 230 in a single two-dimensional radiographic image.

The devices, systems, and methods disclosed herein include confirming and/or observing various stages of deployment of the system in an aneurysm, including complete or substantially complete deployment, using one, some, or all of the methods disclosed above.

### IV. Selected Embodiments

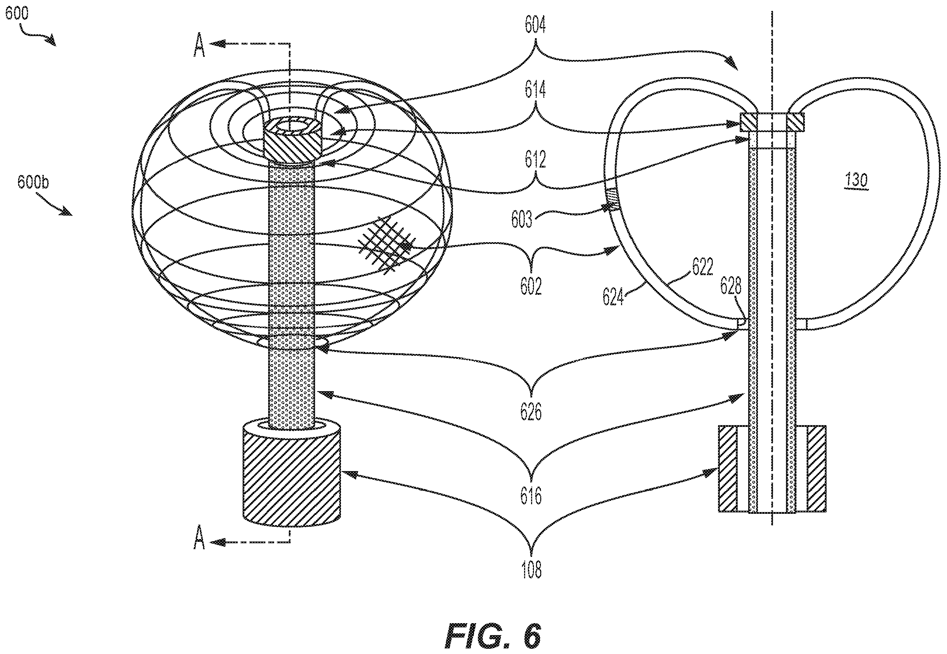

FIG. 6 shows the distal portion of a treatment system 600 for treating an aneurysm in accordance with the present technology. The treatment system 600 may be a portion of a system that also includes an embolic kit (not shown). The embolic kit may be substantially the same as the embolic kit 200 described above. As shown in FIG. 6, the treatment system 600 may comprise an occlusive member 602 (shown in an expanded state) detachably coupled to a delivery system, and the delivery system may be configured to intravascularly position the occlusive member 602 within an aneurysm. In some embodiments, the treatment system 600 may be configured to deliver the embolic element (and/or one or more precursors thereof) to the aneurysm. Additionally or alternatively, the system may include a separate delivery system (not shown) for delivering the embolic element (and/or one or more precursors thereof) to the aneurysm cavity.

Similar to treatment system 100, the treatment system 600 has a proximal portion (not shown) configured to be extracorporeally positioned during treatment and a distal portion 600b configured to be intravascularly positioned within a blood vessel (such as an intracranial blood vessel) at a treatment site at or proximate an aneurysm. The treatment system 600 may include a handle at the proximal portion, the occlusive member 602 at the distal portion 600b, and a plurality of elongated shafts or members extending between the proximal and distal portions. In some embodiments, such as that shown in FIG. 6, the treatment system 600 may include a first elongated shaft (such as a guide catheter or balloon guide catheter) (not depicted), a second elongated shaft 108 (such as a microcatheter) configured to be slidably disposed within a lumen of the first elongated shaft, and an elongated member 616 configured to be slidably disposed within a lumen of the second elongated shaft 108. In contrast to the delivery system of treatment system 100, the elongated member 616 also functions as the conduit. As such, the treatment system 600 does not include a separate elongated member 616 and conduit. In some embodiments, the treatment system 600 does not include the first elongated shaft and only includes the second elongated shaft 108.

A distal end of the occlusive member 602 may be detachably coupled to a distal end of the elongated member 616. For example, the elongated member 616 may include a first coupler 612 at its distal end, and the occlusive member 602 may include a second coupler 614 configured to detachably couple with the first coupler 612. The elongated member 616 may be configured to deliver the embolic element (and/or one or more precursors thereof) through one or more components of the delivery system (e.g., the first or second elongated shafts 109, 108) to a position at the exterior of the occlusive member 602. As such, the embolic element may be positioned between the occlusive member 102 and an inner wall of the aneurysm cavity, as described in greater detail below.

According to some embodiments, the second elongated shaft 108 is generally constructed to track over a conventional guidewire in the cervical anatomy and into the cerebral vessels associated with the brain and may also be chosen according to several standard designs that are generally available. Accordingly, the second elongated shaft 108 can have a length that is at least 125 cm long, and more particularly may be between about 125 cm and about 175 cm long. In some embodiments, the second elongated shaft 108 may have an inner diameter of about 0.0381 cm (0.015 inches), 0.043 cm (0.017 inches), about 0.053 cm(0.021 inches), or about 0.069 cm(0.027 inches). Other designs and dimensions are contemplated.

The elongated member 616 can be movable within the first and/or second elongated shafts to position the occlusive member 602 at a desired location. The elongated member 616 can be sufficiently flexible to allow manipulation, e.g., advancement and/or retraction, of the occlusive member 602 through tortuous passages. Tortuous passages can include, for example, catheter lumens, microcatheter lumens, blood vessels, urinary tracts, biliary tracts, and airways. The elongated member 616 can be formed of any material and in any dimensions suitable for the task(s) for which the system is to be employed. In some embodiments, the elongated member 616 may comprise any other suitable form of shaft such as an elongated tubular shaft.

In some embodiments, the elongated member 616 can comprise stainless steel, nitinol, cobalt-chrome, or other metal or alloy. In some embodiments, the elongated member 616 can be surrounded over some or all of its length by a coating, such as, for example, polytetrafluoroethylene. The elongated member 616 may have a diameter that is generally constant along its length, or the elongated member 616 may have a diameter that tapers radially inwardly, along at least a portion of its length, as it extends in a distal direction.

### C. Selected Examples of Occlusive Members

Referring still to FIG. 5, the occlusive member 602 may comprise an expandable element having a low-profile or constrained state while positioned within a catheter (such as the second elongated shaft 108) for delivery to the aneurysm and an expanded state in which the expandable element is configured to be positioned within an aneurysm (such as a cerebral aneurysm). According to some embodiments, the occlusive member 602 may comprise a mesh formed of a plurality of braided filaments that have been heat-set to assume a predetermined shape enclosing an interior volume 130 when the mesh is in an expanded, unconstrained state. Example shapes include a globular shape, such as a sphere, a prolate spheroid, an oblate spheroid, and others. As depicted in the cross-sectional view in FIG. 6, the mesh may have inner and outer layers 622, 624 that have distal ends fixed relative to one another at the second coupler 614 and meet distally at a proximal fold 628 surrounding a proximal aperture 626. While the inner and outer layers 622, 624 are depicted spaced apart from one another along their lengths, the inner and outer layers 622, 624 may be in contact with one another along all or a portion of their lengths. For example, the inner layer 622 may press radially outwardly against the outer layer 624. In some embodiments, the occlusive member 602 may be formed of a single layer or mesh or braid.

The elongated shaft 616 may be configured to be slidably positioned through some or all of the second coupler 614, the interior volume 130 of the expanded mesh, and the opening 626. Because the occlusive member 602 is coupled to the elongated shaft 616 at its distal end (e.g., via the first and second couplers 612, 614), axial movement of the elongated shaft 616 causes axial movement of at least the distal portion of the occlusive member 602. The proximal portion of the occlusive member 602 remains free to slide along the elongated shaft 616 via the proximal aperture 626. In some embodiments, when the occlusive member 602 is loaded in the elongated member 616, the occlusive member 602 is positioned around the elongated member 616, between the elongated member 616 and the second elongated shaft 108.

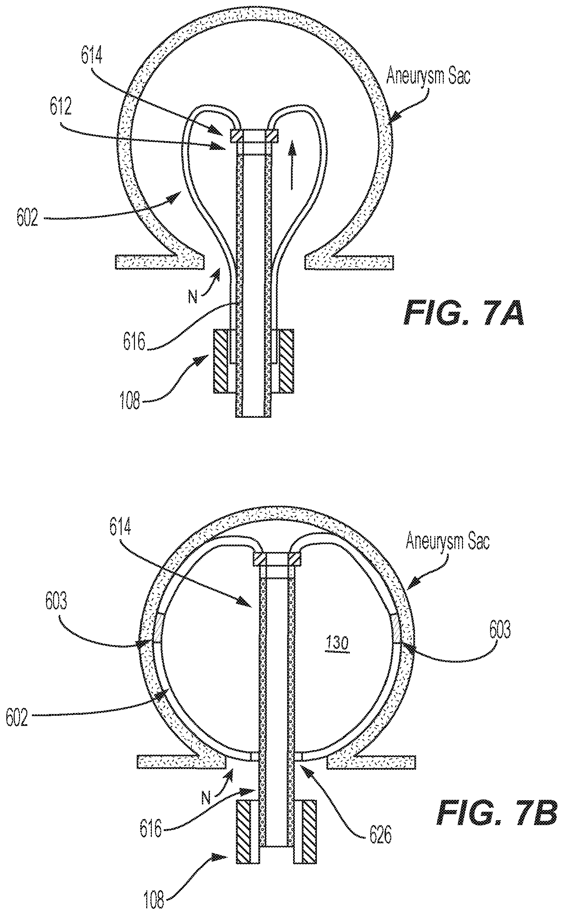

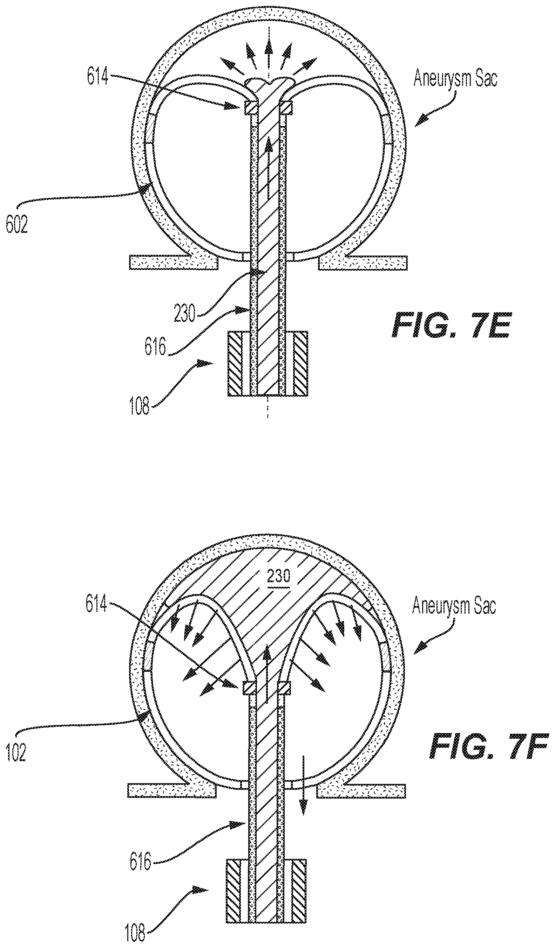

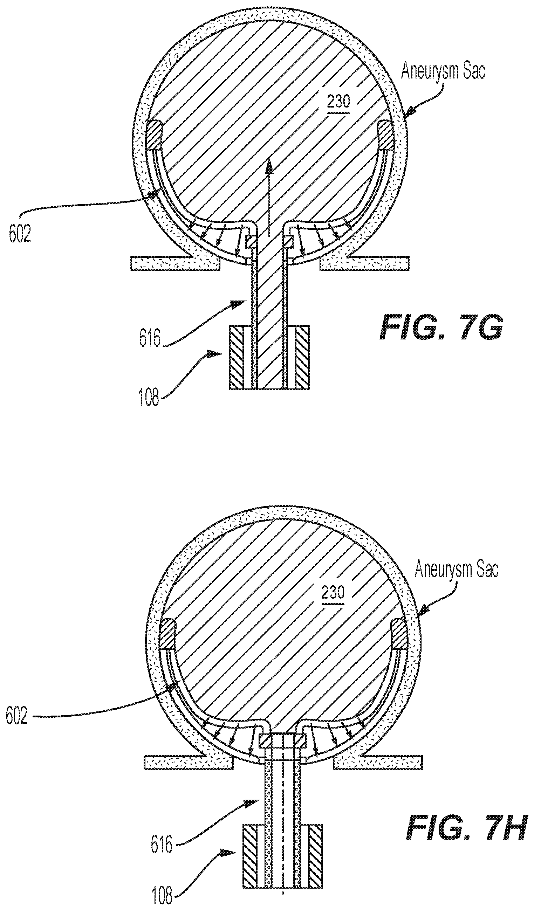

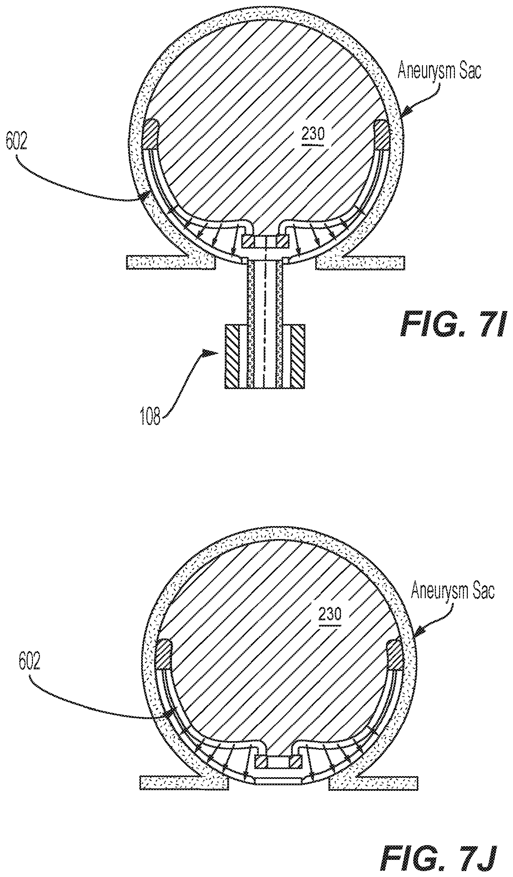

The inner and outer layers 622 and 624 may conform to one another at the distal portion (for example as shown in FIG. 5) to form a curved distal surface. For example, at least at the distal portion of the occlusive member 602, the inner and outer layers 622 and 624 may extend distally and radially inwardly, towards the aperture 626. In some embodiments, the outer and/or inner layers 622 and 624 extend distally and radially outwardly from the fold 628 at the aperture 626, then extend distally and radially inwardly up to ridge, then proximally to the second coupler 614 to create a detent or recess 604 at the distal end of the occlusive member 602. For example, the distal surface of the occlusive member 602 may be concave towards the dome of the aneurysm. The distal recess 604 may be beneficial for delivering the embolic element 230 in that it creates a small gap between the distal surface of the occlusive member 602 and the dome of the aneurysm A (see, for example, FIG. 7B).

The occlusive member 602 and/or layers thereof may be curved along its entire length, or may have one or more generally straight portions. In some embodiments, the curved surface transitions to a flat or substantially flat, distal-most surface that surrounds the aperture 626. In some embodiments, the curved surface transitions to a distal-most surface that surrounds the aperture 626 and has a radius of curvature that is greater than the average radius of curvature of the rest of the occlusive member 602. Having a flat or substantially flat distal surface, or a distal surface with a radius of curvature that is greater than the average radius of curvature of the rest of the occlusive member 602, may be beneficial for delivering the embolic element 230 in that it creates a small gap between the distal surface of the occlusive member 602 and the dome of the aneurysm A. In some embodiments, the surface of the occlusive member 602 surrounding the aperture 626 is curved and/or has generally the same radius of curvature as the remainder of the occlusive member 602.

In any case, the inner layer 624 may have a shape that substantially conforms to the shape of the outer layer 624, or the inner and outer layers 622, 624 may have different shapes. For example, the inner layer 622 may have a diameter or cross-sectional dimension that is less than the outer layer 624 (for example, as shown in FIG. 1D). Such a configuration may be beneficial in that the embolic element 230 experiences less resistance, at least initially, when pushing the distal wall of the occlusion member 602 downwardly towards the neck (as described in greater detail below).

In any case, both the proximal portion and the distal portion of the mesh can form generally closed surfaces. However, unlike at the second coupler 614 at the distal portion of the mesh, the portion of the filaments at or near the fold 628 at the proximal portion of the mesh can move relative to one another. As such, the distal portion of the mesh has both the properties of a closed end and also some properties of an open end (like a traditional stent), such as some freedom of movement of the proximal-most portions of the filaments and an opening through which the elongated shaft 616, a guidewire, guidetube, or other elongated member may pass through.

In some embodiments, each of the plurality of filaments have a first end positioned at the distal portion of the mesh and a second end also positioned at the distal portion of the mesh (for example, at the second coupler 614). Each of the filaments may extend from its corresponding first end distally to ridge, then proximally along the body of the mesh to the fold 628, invert, then extend distally along the mesh body to the ridge, then proximally to its corresponding second end at the distal portion of the mesh. As such, each of the plurality of filaments have a first length that forms the inner layer 622 of the mesh, a second length that forms the outer layer 624 of the mesh, and both first and second ends fixed at the distal portion of the mesh. In some embodiments, the occlusive member 602 may comprise a mesh formed of a single layer, or a mesh formed of three or more layers.

In some embodiments, the proximal and/or distal end surface of the mesh is completely closed (i.e., does not include an aperture). In some embodiments the filaments are fixed relative to the at both the proximal and distal ends of the occlusive member 602 and the elongated member 616 is configured to be slidably disposed through each of the proximal and distal hubs.

In some embodiments, the occlusive member 602 and/or mesh may include a preferential bending region 604 at which the occlusive member 602 and/or mesh is configured to preferentially flex or bend when subject to inverting forces (such as that exerted by the embolic element 230, as described in greater detail below). The bending region 604 may form a continuous band around the circumference of the occlusive member 602, or it may be at select locations about the circumference of the occlusive member 602, all located at generally the same axial location along the mesh. In those embodiments in which the occlusive member 602 comprises multiple layers, none, some, or all of the layers may include the bending region 604. The bending region 604 may comprise, for example, a thinned portion of the mesh that is generally weaker than the surrounding portions and thus more likely to bend under stress. The bending region 604 may also be formed by heat setting the mesh in the inverted or collapsed state such that the mesh preferentially inverts at the desired level.

The mesh forming the occlusive member 602 may be formed of metal wires, polymer wires, or both, and the wires may have shape memory and/or superelastic properties. The mesh may be formed of 24, 32, 36, 48, 64, 72, 96, 128, or 144 filaments. The mesh may be formed of a range of filament or wire sizes, such as wires having a diameter of from about 0.0102 mm (0.0004 inches) to about 0.0508 mm (0.0020 inches), or of from about 0.0229 mm (0.0009 inches) to about 0.0305 mm (0.0012 inches). In some embodiments, each of the wires or filaments have a diameter of about 0.0102 mm (0.0004 inches), about 0.0127 mm (0.0005 inches), about 0.0152 mm (0.0006 inches), about 0.0178 mm (0.0007 inches), about 0.0203 mm (0.0008 inches), about 0.0229 mm (0.0009 inches), about 0.0254 mm (0.001 inches), about 0.0279 mm (0.0011 inches), about 0.0305 mm (0.0012 inches), about 0.0330 mm (0.0013 inches), about 0.0356 mm (0.0014 inches), about 0.0381 mm (0.0015 inches), about 0.0406 mm (0.0016 inches), about 0.0432 mm (0.0017 inches), about 0.0457 mm (0.0018 inches), about 0.0483 mm (0.0019 inches), or about 0.0508 mm (0.0020 inches). In some embodiments, all of the filaments of the braided mesh may have the same diameter. For example, in some embodiments, all of the filaments have a diameter of about 0.025 mm (0.001 inches). In some embodiments, some of the filaments may have different cross-sectional diameters. For example, some of the filaments may have a slightly thicker diameter to impart additional strength to the braided layers. In some embodiments, some of the filaments can have a diameter of about 0.025 mm (0.001 inches), and some of the filaments can have a diameter of greater than 0.025 mm (0.001 inches). The thicker filaments may impart greater strength to the braid without significantly increasing the device delivery profile, with the thinner wires offering some strength while filling-out the braid matrix density.

The occlusive member 602 can have different shapes and sizes in an expanded, unconstrained state. For example, the occlusive member 602 may have a bullet shape, a barrel-shape, an egg shape, a dreidel shape, a bowl shape, a disc shape, a cylindrical or substantially cylindrical shape, a barrel shape, a chalice shape, etc.

### D. Selected Examples of Embolic Kits

The embolic kit for use with the treatment system shown in FIGS. 6-7J may be substantially similar to the embolic kit 200 described above.

### E. Selected Methods of Deployment

FIGS. 7A-7J depict an example method of treating an aneurysm with the treatment systems of the present technology. To begin, a physician may intravascularly advance the second elongated shaft 108 towards an intracranial aneurysm (or other treatment location such as any of those described herein) with the occlusive member 602 in a low-profile state. As shown in FIG. 7A, the second elongated shaft 108 may be positioned at or just proximal of the neck. Alternatively, a distal portion of the second elongated shaft 108 may be advanced through a neck N of the aneurysm A to locate a distal opening of the second elongated shaft 108 within an interior cavity of the aneurysm A. In either case, the elongated member 616 may be advanced distally through the neck N and into the aneurysm cavity (if not already in the aneurysm cavity), thereby pulling the distal end of the occlusive member 602 with it. As the occlusive member 602 is released from its constrained state within the second elongated shaft 108, the occlusive member 602 expands towards the first expanded state, as shown in FIG. 7B. In the first expanded state, the occlusive member 602 may assume a predetermined shape that encloses an internal volume 130. In this first expanded state, the occlusive member 602 may generally conform to the shape of the aneurysm A.

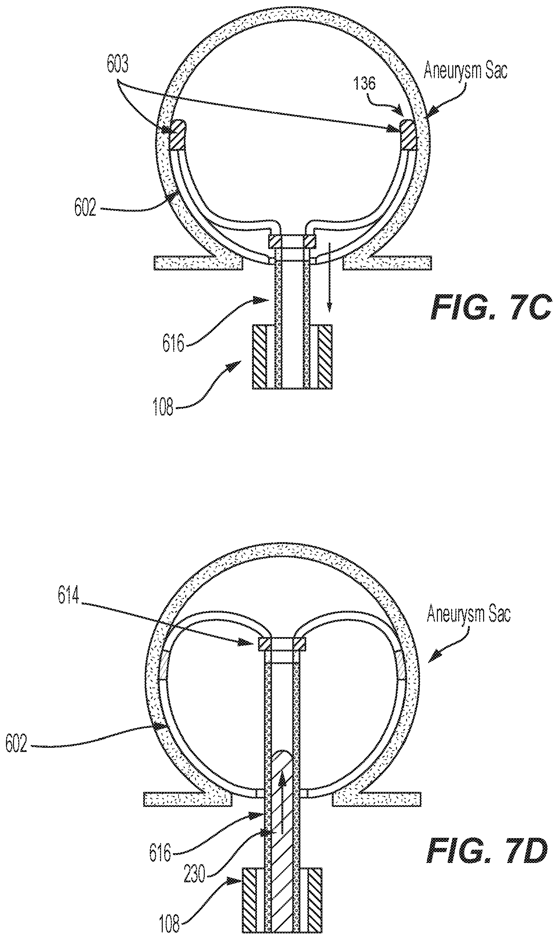

Prior to delivering the embolic agent 230, the physician may pull the elongated member 616 proximally to check the positioning of the occlusive member 602 in the aneurysm A. As depicted in FIGS. 7B and 7C, for example, the elongated member 616 may be pulled proximally to force the distal wall towards the neck N and proximal wall. Because of the bending region(s) 603, the occlusive member 602 may preferentially fold and develop a distal edge at the bending regions 603. The ability to check the position of the occlusive member 602 in its collapsed state before delivering the embolic element 230 may be beneficial, especially in wide-necked aneurysms, as the initial approach into the aneurysm may be too steep such that the distal edge 136 (see FIG. 7C), once the occlusive member 602 is fully collapsed or inverted, is near or aligned with the neck of the aneurysm A. In those cases, the occlusive member 602 may not fully cover the neck N, and thus will not be sufficiently anchored in the aneurysm A. It will be appreciated that the elongated member 616 may be used to check the positioning of the occlusive member 602 even in those embodiments where the occlusive member 602 does not have a bending region 603.

As shown in FIGS. 7D-7H, the embolic element 230 may be delivered through the elongated member 616 to a space between the occlusive member 602 and an inner surface of the aneurysm wall W. In some embodiments, the method includes mixing the first and second precursor materials 203, 205 (FIG. 2) to form the embolic element 230. Mixing of the first and second precursor materials 203, 205 may occur prior to introducing the embolic element 230 to the treatment system 100 and/or during delivery of the embolic element through the elongated member 616 to the aneurysm. In a particular example, the first precursor material 203 is loaded into one of the barrels 214, the second precursor materials 205 is loaded into the other barrel 214, and the mixing syringes 208 are coupled via the coupler 210. To mix the first and second precursor materials 203, 205, the plungers 212 are alternately depressed, thereby causing the first and second precursor materials 203, 205 to move repeatedly from one barrel 214 to the other barrel 214. After suitably mixing the precursor materials, the resulting embolic element 230 can be loaded into the barrel 220 of the injection syringe 216. The injection syringe 216 may then be coupled to a proximal end of the elongated member 616 to deliver the embolic element 230 through the elongated member 616 and into the aneurysm A. As the embolic element 230 passes through the lumen of the elongated member 616, chemical crosslinking of the biopolymer can continue to occur.

Still with reference to FIGS. 7D-7H, as the embolic element 230 is delivered between the dome of the aneurysm A and the distal portion of the wall of the occlusive member 602, pressure builds between the aneurysm wall W and the occlusive member 602. When the forces on the occlusive member 602 reach a threshold level, the embolic element 230 pushes the distal wall downwardly towards the neck N of the aneurysm A. If desired, the elongated member 616 may be pulled proximally while the embolic element 230 is delivered and/or between different injections of the embolic element 230, which encourages inversion of the occlusive member 602.

As detailed above, the embolic element 230 exerts a substantially uniform pressure across the distal surface of the occlusive member 602 that collapses the occlusive member 602 inwardly on itself (with or without the help of the elongated member 616). The pressure and inversion of the distal portion of the wall creates an annular fold 136 that defines the distal-most edge of the occlusive member 602. As the occlusive member 602 continues to invert, the position of the fold moves towards the neck N, which continues until a distal-most half of the occlusive member 602 has inverted. Moreover, as the occlusive member 602 collapses, a distance between the wall at the distal portion and the wall at the proximal portion decreases, and thus the internal volume 130 of the occlusive member 602 also decreases. As the occlusive member 602 collapses, the elongated member 616 may be held stationary, advanced distally, and/or retracted proximally.

During and after delivery of the embolic element 230, none or substantially none of the embolic element 230 migrates through the pores of the occlusive member 602 and into the internal volume 130. Said another way, all or substantially all of the embolic element 230 remains at the exterior surface or outside of the occlusive member 602. Compression of the occlusive member with the embolic element 230 provides a real-time "leveling" or "aneurysm-filling indicator" to the physician under single plane imaging methods (such as fluoroscopy) so that the physician can confirm at what point the volume of the aneurysm is completely filled. Additional details regarding devices, systems, and methods for monitoring and/or confirming deployment are described above with reference to FIGS. 4A-5B. It is beneficial to fill as much space in the aneurysm as possible, as leaving voids within the aneurysm sac may cause delayed healing and increased risk of aneurysm recanalization and/or rupture. While the scaffolding provided by the occlusive member 602 across the neck helps thrombosis of blood in any gaps and healing at the neck, the substantial filling of the cavity prevents rupture acutely and does not rely on the neck scaffold (i.e., the occlusive member 602). Confirmation of complete or substantially complete aneurysm filling under single plane imaging cannot be provided by conventional devices.

Once delivery of the embolic element 230 is complete, the elongated member 616 may be withdrawn. In some embodiments, the embolic element 230 may fill greater than 40% of the aneurysm sac volume. In some embodiments, the embolic element 230 may fill greater than 50% of the aneurysm sac volume. In some embodiments, the embolic element 230 may fill greater than 60% of the aneurysm sac volume. In some embodiments, the embolic element may fill greater than 65%, 70%, 75%, 80%, 85%, or 90% of the aneurysm sac volume.

FIG. 7I shows a second expanded state of the occlusive member 602, shown in cross-section, with the embolic element 230 occupying the remaining volume of the aneurysm A. As shown, the embolic element 230 may be delivered until the occlusive member 602 is fully-collapsed such that the occlusive member 602 has substantially no internal volume. In the second expanded state, the occlusive member 602 may form a bowl shape that extends across the neck of the aneurysm A. The wall of the occlusive member 602 at the distal portion may now be positioned in contact with or immediately adjacent the wall of the occlusive member 602 at the proximal portion. In some instances, because of the presence of the second coupler 614, a small gap may remain between portions of the distal wall and the proximal wall when the occlusive member 602 is in its second or collapsed state.

Collapse of the occlusive member 602 onto itself, towards the neck N of the aneurysm, may be especially beneficial as it doubles the number of layers across the neck and thus increases occlusion at the neck N. For example, the distal wall 132 collapsing or inverting onto the proximal wall may decrease the porosity of the occlusive member 602 at the neck N. In those embodiments where the occlusive member 602 is a mesh or braided device such that the distal wall has a first porosity and the proximal wall has a second porosity, deformation of the distal wall onto or into close proximity within the proximal wall decreases the effective porosity of the occlusive member 602 over the neck N. The resulting multi-layer structure thus has a lower porosity than the individual first and second porosities. Moreover, the embolic element 230 along the distal wall provides additional occlusion. In some embodiments, the embolic element 230 completely or substantially completely occludes the pores of the adjacent layer or wall of the occlusion member 602 such that blood cannot flow past the embolic element 230 into the aneurysm cavity. It is desirable to occlude as much of the aneurysm as possible, as leaving voids of gaps can allow blood to flow in and/or pool, which may continue to stretch out the walls of aneurysm A. Dilation of the aneurysm A can lead to recanalization and/or herniation of the occlusive member 602 and/or embolic element 230 into the parent vessel and/or may cause the aneurysm A to rupture. Both conditions can be fatal to the patient.

In those embodiments where the wall of the occlusive member 602 comprises an inner and outer layer, the deformed or second shape of the occlusive member 602 forms four layers over the neck N of the aneurysm A In those embodiments where the wall of the occlusive member 602 comprises a single layer, the deformed or second shape of the occlusive member 602 forms two layers over the neck N of the aneurysm A As previously mentioned, the neck coverage provided by the doubled layers provides additional surface area for endothelial cell growth, decreases the porosity of the occlusive member 602 at the neck N (as compared to two layers or one layer), and prevents herniation of the embolic element 230 into the parent vessel. During and after delivery, the embolic element 230 exerts a substantially uniform pressure on the occlusive member 602 towards the neck N of the aneurysm A, thereby pressing the portions of the occlusive member 602 positioned adjacent the neck against the inner surface of the aneurysm wall such that the occlusive member 602 forms a complete and stable seal at the neck N.

In some embodiments, the elongated member 616 may be moved proximally and distally to move the distal wall of the occlusive member 602 to re-distribute the embolic element 230 around the occlusive member 602 and the aneurysm wall. As a result, the occlusive member 602 will be locked and prevent migration after treatment. In this case, the occlusive member 602 may not need to fill the sac completely.

As shown in FIGS. 7I-7J, the first coupler 612 may be detached from the second coupler 614 and the elongated member 616 and second elongated shaft 108 may be withdrawn, thereby leaving the occlusive member 602 and embolic element 230 implanted within the aneurysm A.

Over time natural vascular remodeling mechanisms and/or bioabsorption of the embolic element 230 may lead to formation of a thrombus and/or conversion of entrapped thrombus to fibrous tissue within the internal volume of the aneurysm A. These mechanisms also may lead to cell death at a wall of the aneurysm and growth of new endothelial cells between and over the filaments or struts of the occlusive device 602. Eventually, the thrombus and the cells at the wall of the aneurysm may fully degrade, leaving behind a successfully remodeled region of the blood vessel.

In some embodiments, contrast agent can be delivered during advancement of the occlusive member 602 and/or embolic element 230 in the vasculature, deployment of the occlusive member 602 and/or embolic element 230 at the aneurysm A, and/or after deployment of the occlusive member 602 and/or embolic element 230 prior to initiation of withdrawal of the delivery system. The contrast agent can be delivered through the second elongated shaft 108, the elongated member 616, or through another catheter or device commonly used to delivery contrast agent. The aneurysm (and devices therein) may be imaged before, during, and/or after injection of the contrast agent, and the images may be compared to confirm a degree of occlusion of the aneurysm.

### V. Example Systems with Electrolytic Detachment Mechanisms

### A. Detachment via Electrolytically Corrodible Core Wire

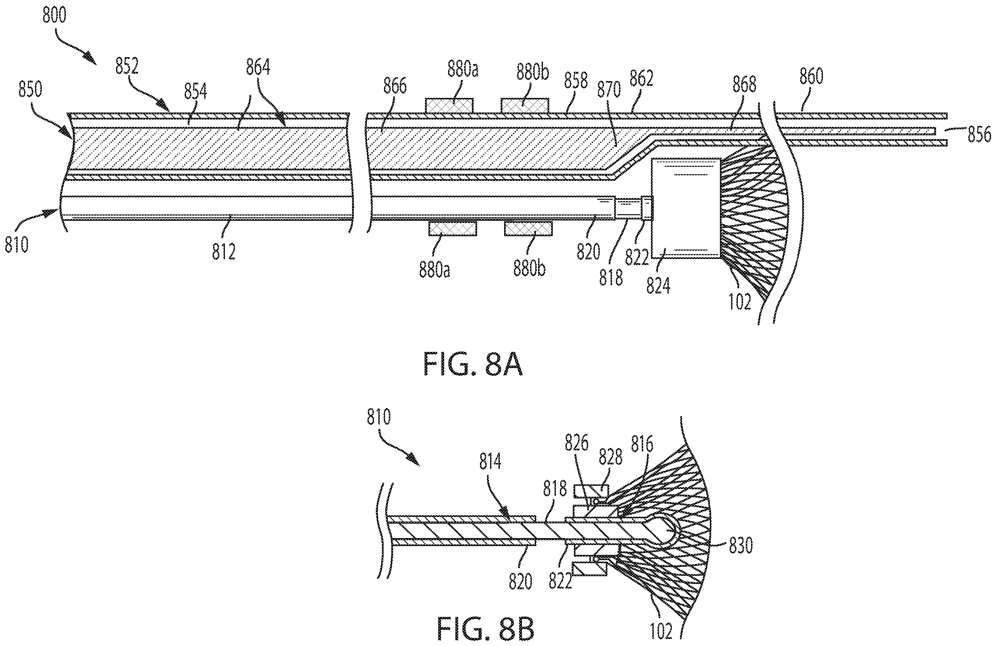

FIG. 8A shows a schematic side view of a treatment system 800, and FIG. 8B shows a side cross-sectional view of a distal portion of the treatment system 800 shown in FIG. 8A, with the occlusive member delivery assembly omitted for clarity. As described in more detail below, the treatment system 800 can include an occlusive member delivery assembly 810 and an embolic element delivery assembly 850, which can be coupled together via one or more couplers 880. In operation, the occlusive member delivery assembly 810 facilitates placement of the occlusive member 102 at the treatment site and utilizes electrolytic detachment to release the occlusive member 102 from its delivery assembly 810. The embolic element delivery assembly 850, which can extend adjacent and generally parallel to the occlusive member delivery assembly 810 along some or all of its length, can facilitate introduction of an embolic element 230 (FIGS. 2-3G) therethrough for placement at the treatment site.

The occlusive member delivery assembly 810 includes the occlusive member 102 coupled to a distal end of the elongated member 106. In some embodiments, the elongated member 106 can take the form of an electrolytically corrodible core wire 812, which may be monolithic or composed of multiple separate components joined together. According to some embodiments, as shown in FIGS. 8A and 8B, the electrolytically corrodible core wire 812 includes a proximal portion 814, a distal portion 816, and a detachment zone 818 disposed between the proximal portion 814 and the distal portion 816. At least a portion of the core wire 812, including the detachment zone 818, can be coated with a conductive material, such as carbon, gold, platinum, tantalum, combinations thereof, and the like. One or more metallic coatings can be applied using known plating techniques.

The core wire 812, including the detachment zone 818, can include one or more of the following materials: ceramic materials, plastics, base metals or alloys thereof, and preferably stainless steel. Some of the most suitable material combinations for forming the electrolytically corrodible points can include one or more of the following: stainless steels, preferably of the type AISI 301, 304, 316, or subgroups thereof; Ti or TiNi alloys; Co-based alloys; noble metals; or noble metal alloys, such as Pt, Pt metals, Pt alloys, Au alloys, or Sn alloys. Further, ceramic materials and plastics employed for forming the medical device can be electrically conductive.

According to some embodiments, portions of the core wire 812 can be coated with a nonconductive material. A proximal insulating layer 820 can be provided over at least a portion of an outer surface of the proximal portion 814 of the core wire 812. For example, the proximal insulating layer 820 can circumferentially surround an outer surface of the proximal portion 814. A distal insulating layer 822 can be provided over at least a portion of an outer surface of the distal portion 816 of the core wire 812. For example, the distal insulating layer 822 can circumferentially surround and contact an outer surface of the distal portion 816. The proximal and distal insulating layers 820, 822, can be of an electrically nonconductive or insulative polymer, such as polyimide, polypropylene, polyolefins, combinations thereof, and the like.

According to some embodiments, proximal and distal insulating layers 820, 822 leave exposed the detachment zone 818 of the core wire 812. When in contact with a body fluid, such as blood, the fluid serves as an electrolyte allowing current to be focused on the non-coated detachment zone 818. The proximal and distal insulating layers 820, 822 prevent exposure of the proximal portion 814 and distal portion 816 to the fluid. Accordingly, electrical energy conducted along the core wire 812 is concentrated at the detachment zone 818, thereby reducing the time required to erode away the detachment zone 818. The proximal and distal insulating layers 820, 822 can be over-molded, co-extruded, sprayed on, or dip-coated with respect to the core wire 812.

Laser ablation can be employed to selectively remove the coating to a controlled length minimizing the time required to erode through the component. Lengths as small as 0.0127mm (0.0005") and as large as 2.54mm (0.1") or longer can be removed. According to some embodiments, lengths of detachment zone 818 can be greater than 0.0127mm (0.0005") and/or less than 0.254mm (0.010") to provide sufficient exposure to achieve detachment times of less than 30 seconds. In some embodiments, the detachment zone 818 can have a smaller cross-sectional profile or outer diameter than the proximal and/or distal portions 814, 816. In some embodiments, the detachment zone 818 can include additional or alternative materials to facilitate electrolytic corrosion at this zone.

According to some embodiments, the distal insulating layer 822 is disposed radially between the distal portion 816 of the core wire 812 and the hub 824 of the occlusive member 102. As shown in FIG. 8B, an inner band 826 of the hub 824 circumferentially surrounds and contacts the distal insulating layer 822. An outer band 828 surrounds the inner band 826, such that proximal portions of the layers of the occlusive member 102 are grasped between the inner and outer bands 826, 828 of the hub 824.

As shown in FIG. 8B, the distal insulating layer 822 electrically isolates the occlusive member 102 from an electrical charge conducted along a length of the core wire 812. A proximal end of the distal insulating layer 822 may be positioned proximal to the hub 824, and a distal end of the distal insulating layer 822 may be positioned distal to the hub 824. Likewise, a proximal end of the distal portion 816 may be positioned proximal to the hub 824, and a distal end of the distal portion 816 may be positioned distal to the hub 824, such that the distal portion 816 extends through and distally beyond a lumen formed by the hub 824.

The core wire 812 can include an anchor end 830 at a terminal distal end of the core wire 812. The anchor end 830 can be located distal to the hub 824. For example, the anchor end 830 can be located within an interior portion of the occlusive member 102. The anchor end 830 can have a maximum cross-sectional dimension that is greater than an inner cross-sectional dimension of the inner band 826. Accordingly, the core wire 812 is prevented from moving proximally entirely through the inner band 826. For example, an interface between the distal insulating layer 822 and the inner band 826 or an interface between the distal insulating layer 822 and the core wire 812 may allow a degree of movement of the core wire 812 relative to the inner band 826. To prevent the core wire 812 from being removed distally from within the inner band 826, the anchor end 27 can be of a size that cannot pass entirely proximally through the inner band 826.

Alternatively, the proximal end of the distal insulating layer 822 may be coterminous with a proximal end of the hub 824, and/or a distal end of the distal insulating layer 822 may be coterminous with a distal end of the hub 824. Likewise, the proximal end of the distal portion 816 may be coterminous with a proximal end of the hub 824, and/or a distal end of the distal portion 816 may be coterminous with a distal end of the hub 824.

According to some embodiments, a detachment zone 818 can be configured such that the corrodible portion thereof defines a unique structure configured to enhance electrolytic corrosion while preserving the structural characteristics thereof. A reduction in corrosion resistance will reduce a time required to deploy an intravascular and/or intrasaccular implant, thus reducing the overall procedure time. According to some embodiments, corrosion resistance of detachment zone 818 is decreased by exposure to laser or other energy, causing the detachment zone 818 to be structurally modified by heat. As a result, the detachment zone 818 will have a different microstructure than the material outside of the zone (e.g., the proximal portion 814 and/or the distal portion 816 of the core wire 812). The result will decrease the time to electrolytically plate off the material, resulting in faster detachment times.

The laser energy will create surface defects for a reduction in corrosion resistance. The laser energy will also alter the microstructure at a specific area, leading to a non-uniform corrosion rate. Accordingly, the preferred corrosion site can have a faster detach time. According to some embodiments, the proximal portion 814 and/or the distal portion 816 have a microstructure with a crystallinity that is greater than a crystallinity of a microstructure of the detachment zone 818. According to some embodiments, the detachment zone 818 comprises a microstructure that is more amorphous than each of (i) a microstructure of the proximal portion 814 and (ii) a microstructure of the distal portion 816. According to some embodiments, a method of treating includes providing an electrolytically corrodible core wire 812 comprising a proximal portion 814, a distal portion 816, and a detachment zone 818 between the proximal portion 814 and the distal portion 816. The detachment zone 818 is treated to produce a microstructure in the detachment zone 818 that is more amorphous than each of (i) a microstructure of the proximal portion 814 and (ii) a microstructure of the distal portion 816.

A shown in FIG. 8A, the embolic element delivery assembly 850 can extend adjacent to the occlusive member delivery assembly 810. In some embodiments, the embolic element delivery assembly 850 includes a conduit 852 defining a lumen 854 extending therethrough. The lumen 854 can terminate in a distal opening 856. In some embodiments, the conduit 852 can be an elongate flexible tubular member, for example a catheter, hypotube, polymer tube, etc. The lumen 854 can be coated with a lubricious material or lining to facilitate advancement of embolic element(s) therethrough. In some embodiments, the conduit 852 is dimensioned such that the distal opening 856 is disposed adjacent to, completely distal of, or at least partially distal of the occlusive member 102 while the occlusive member 102 is in the unexpanded state. In some embodiments, the conduit 852 can be dimensioned and configured such that the distal opening 856 is disposed at distal to the hub 824 of the occlusive member 102, such that embolic element(s) delivered therethrough can be delivered to a region adjacent or distal of the occlusive member 102.

As seen in FIG. 8A, the conduit 852 and its lumen 854 can each have a cross-sectional dimension that varies along the length of the conduit 852. For example, the conduit 852 can include a proximal portion 858, a distal portion 860, and a transition portion 862 between the proximal and distal portions 858, 860. In some embodiments, the proximal portion 858 has a larger cross-sectional dimension than the distal portion 860, and the transition portion 862 can have a tapered profile or diameter. In some embodiments, the transition portion 862 can include one or more steps or abrupt transitions instead of or in addition to any gradual tapering transitions. In the illustrated embodiment, the wall thickness of the conduit 852 is substantially uniform, such that the conduit 852 and the lumen 854 step down in cross-sectional dimension in tandem. In some embodiments, the wall thickness may vary, for example such that while the outer cross-sectional dimension of the conduit 852 steps down according to a first profile, the lumen 854 steps down according to a different profile (e.g., with a more or less gradual taper), or in some instances the lumen 854 may be substantially uniform along its length. In still other embodiments, the conduit 852 can have a substantially constant diameter along its length, or may gradually taper over substantially its entire length.

A stylet 864 can be sized and configured to be slidably received within the lumen 854 of the conduit 852. In operation, the stylet 864 can provide increased rigidity to enhance pushability of the conduit 852, which may otherwise be too flexible to permit pushability through a surrounding guide catheter. In various embodiments, the stylet 864 can be metallic, polymeric, rubber, or any other suitable material. In some embodiments, the stylet 864 is generally stiffer than the conduit 852. As seen in FIG. 8A, the stylet 864 can have an outer cross-sectional dimension that substantially corresponds to the lumen 854 of the conduit 852, for example having a proximal portion 866, a distal portion 868, and a transition portion 870 therebetween. Alternatively, the stylet 864 may have a constant cross-sectional dimension.

As noted previously, the occlusive member delivery assembly 810 and the embolic element delivery assembly 850 can be coupled together via one or more couplers 880. In the embodiment shown in FIG. 8A, the couplers include a first coupler 880a and a second coupler 880b. The couplers 880 can be loops or bands that extend circumferentially around both the conduit 852 and the core wire 812 to secure them together. Such bands can be made of any suitable material, for example being polymeric or metallic, and optionally may be radiopaque to facilitate visualization of the system 800 as it advanced through the vasculature. The bands can be crimped over the assemblies 810, 850, with or without an adhesive or weld to secure them in place. Although two such couplers 880 are shown, the number of couplers can vary, for example one, three, four, five, or more couplers can be used to secure the conduit 852 and the core wire 812 together. In some embodiments, the coupler 880 can take the form of an adhesive that fastens at least a portion of the core wire 812 to at least a portion of the conduit 852. In some embodiments, the coupler 880 can include a surrounding sheath, for example a polymeric material that can surround the conduit 852 and the core wire 812 to securely hold them together, for example being heat-shrunk or otherwise snugly fit over the conduit 852 and the core wire 812. In various embodiments, the occlusive member delivery assembly 810 and the embolic element delivery assembly 850 can be coupled together such that the two are not slidable or rotatable relative to one another. In other embodiments, the two assemblies 810, 850 can be slidably coupled together, such that the embolic element delivery assembly 850 can be slidably advanced or retracted with respect to the occlusive member delivery assembly 810. In other embodiments, the two assemblies 810, 850 may be uncoupled, such that each can be separately advanced to the treatment site simultaneously or sequentially.

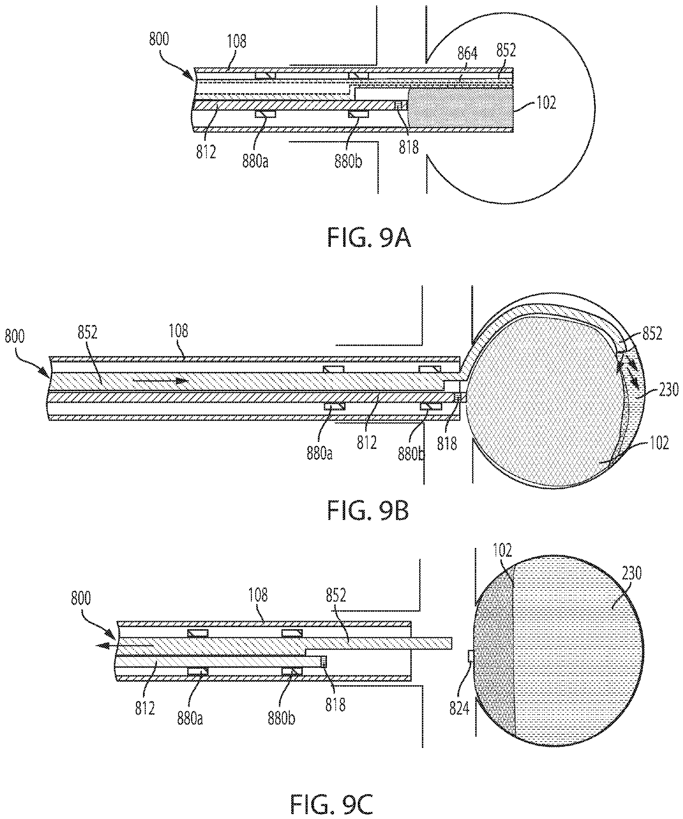

FIGS. 9A-9C illustrate delivery of an occlusive member 102 and embolic element 230 to a treatment site within an aneurysm sac. As shown in FIG. 9A, the system 800 can be positioned within a second elongate shaft 108 (e.g., a microcatheter) for intravascular advancement until the microcatheter is at or adjacent to the aneurysm sac. In the illustrated embodiment, the distal end of the second elongate shaft 108 extends within the aneurysm sac, however in other embodiments the distal end of the second elongate shaft 108 can be positioned at the neck of the aneurysm or proximal to the neck of the aneurysm.

In the position shown in FIG. 9A, the system 800 has been advanced within the elongate shaft 108 such that the occlusive member 102 remains in a constrained, low-profile configuration within the shaft 108 while at least a portion of the conduit 852 extends adjacent to the occlusive member 102 and within the shaft 108. In various embodiments, the shaft 108 can have an inner diameter of about 0.432 mm (0.017 inches) or less, about 0.533 mm (0.021 inches) or less, or about 0.686 mm (0.027 inches) or less.

As shown in FIG. 9B, once the distal opening 856 of the conduit 852 is positioned at or near the treatment site (e.g., within the aneurysm sac), the elongate shaft 108 can be retracted, thereby deploying the occlusive member 102 within the aneurysm sac (e.g., allowing the occlusive member 102 to self-expand). Prior to, concurrently with, or after deployment of the occlusive member 102, the stylet 864 may be removed from within the lumen 854 of the conduit 852. With the stylet 864 removed and the occlusive member 102 expanded, the distal portion of the conduit 852 can assume a curved shape, for example curving along an inner wall of the aneurysm sac and/or along an outer surface of the occlusive member 102. In the illustrated embodiment, the distal opening of the conduit 852 is positioned at a distalmost portion of the aneurysm sac, at the dome of the sac.

In this position, the embolic element 230 can be advanced through the conduit 852 and into the aneurysm to a region distal to the occlusive member 102. In the case of a fluid or gel, a syringe or other injector may be used to urge the embolic element 230 through the lumen 854. In the case of microcoils or other structural embolic element(s), a delivery wire or other suitable mechanism may be slidably advanced through the lumen 854 of the conduit 852 to position the embolic element 230 into the aneurysm sac.

As described previously with respect to FIGS. 3A-3G, introduction of the embolic element 230 can cause the occlusive member 102 to deform, for example to at least partially fold in on itself to provide for increased protection in a neck region of the aneurysm. Once the embolic element 230 been delivered and the occlusive member 102 has deformed, the occlusive member 102 can be severed from the core wire 812 as described above. For example, a power supply or other current source can be used to generate current through the core wire 812, resulting in electrolytic corrosion of the core wire 812 at the detachment zone 818.

As shown in FIG. 9C, after the occlusive member 102 is severed via electrolytic corrosion of the detachment zone 818, the core wire 812 and the embolic element delivery assembly 850 can be proximally retracted while the occlusive member 102 and the embolic element 230 remain positioned within the aneurysm. As the embolic element delivery assembly 850 is retracted, the distal portion of the conduit 852 can slide around the expanded occlusive member 102 and out the neck of the aneurysm.

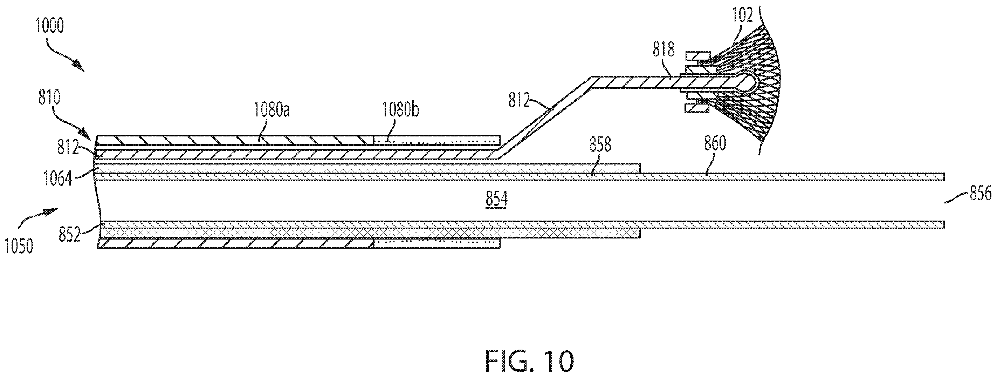

FIG. 10 shows a schematic side view of another embodiment of a treatment system 1000 in accordance with aspects of the present technology. The treatment system 1000 can include several features that are generally similar to those of FIGS. 8A and 8B described above. For example, an occlusive member delivery assembly 810 includes a core wire 812 having a detachment zone 818 positioned proximal to a hub 824 of the occlusive member 102. The core wire 812 can be coated with an insulative material along some or all of its length (with at least the detachment zone 818 being uncovered by the insulative material).

The core wire 812 runs generally parallel and adjacent to an embolic element delivery assembly 1050. This assembly 1050 can include several features similar to those described above with respect to FIGS. 8A and 8B, for example having a conduit 852 defining a lumen 854 extending therethrough. In the illustrated embodiment, the conduit 852 can take the form of an extruded polymeric tube (e.g., PTFE) or other suitable material. The conduit 852 can be sufficiently lubricious to facilitate slidable advancement of embolic element(s) therethrough. The conduit 852 can include a proximal portion 858 and a distal portion 860. In the illustrated embodiment, the conduit 852 has a substantially uniform diameter or cross-sectional dimension along its length, and the lumen 854 likewise has a substantially uniform diameter or cross-sectional dimension along its length. In some embodiments, the cross-sectional dimensions of the lumen 854 or the outer surface of the conduit 852 may vary along their lengths.

In contrast to the stylet 864 of FIG. 8A, the system 1000 shown in FIG. 10 relies on a surrounding reinforcement member 1064 that surrounds at least the proximal portion 858 of the conduit 852. The reinforcement member 1064 can be, for example a hypotube, catheter, or other suitable tubular member having sufficient rigidity, hoop strength, and/or other structural characteristics to allow pushability of the assembly 1050 through a surrounding delivery catheter without the need for a stylet. Because the distal portion 860 of the conduit 852 is not surrounded by the reinforcement member 1064, the distal portion 860 can be more flexible than other portions of the assembly 1050. This can facilitate positioning the distal opening 856 of the conduit 852 at the treatment site (e.g., as shown in FIG. 9B). In some embodiments, the reinforcement member 1064 can extend substantially the entire length of the conduit 852.

As shown in FIG. 10, the core wire 812 of the occlusive member delivery assembly 810 is coupled together to the embolic element delivery assembly via first and second couplers 1080a and 1080b. In contrast to the discrete bands illustrated in FIG. 8A, the first coupler 1080a shown in FIG. 6 can take the form of an elongated sleeve that circumferentially surrounds both the reinforcement member 1064 and the core wire 812 along at least a portion of their respective lengths. The first coupler 1080a can be, for example, a tightly fitting polymeric sheath, for example PTFE that has been heat-shrunk into position over the reinforcement member 1064 and the core wire 812. Distal to the first coupler 1080a is the second coupler 1080b, which can take the form of another elongate sleeve circumferentially surrounding the reinforcement member 1064 and the core wire 812. The second coupler 1080b can be a more flexible polymeric or other suitable material, for example polyether block amide (PEBA), a thermoplastic polyurethane (PTU), or any other suitable material. The more flexible second coupler 1080b may serve to better tolerate relative movement of the portion of the core wire 812 that extends away from the reinforcement member 1064, as the movement of the core wire 812 can exert an outward force on the second coupler 1080b. This can be particularly useful during a resheathing procedure, in which a partially or fully deployed occlusion member 102 is retracted back into a surrounding catheter, which can exert relatively high forces on the core wire 812 and the second coupler 1080b. In some embodiments, the two couplers 1080a and 1080b can be replaced with a single coupler made of any suitable material, or additional couplers may be used.

Although the embodiment of FIG. 10 illustrates the core wire 812 as extending proximally along an outer surface of the reinforcement member 1064, in some embodiments, a proximal portion of the core wire 812 can be replaced by or integrated into the reinforcement member 1064. For example, in the case of a hypotube as the reinforcement member 1064, the hypotube can be configured to carry electrical current along its length to a distally coupled core wire 812 that extends away from the hypotube and is coupled to the occlusive member 102. In another embodiment, the reinforcement member 1064 can include a braided catheter, and electrical current may be carried by one or more conductors extending within the wall of the catheter and in electrical communication with the core wire 812.

The system 1000 shown in FIG. 10 can be used similar to the method illustrated in FIGS. 9A-9C, except that the system 1000 does not include a stylet to be removed after initial positioning of the distal opening 856 of the conduit 852 at the treatment site. As such, the system 1000 can be advanced through the vasculature until the occlusive member 102 and the distal opening 856 of the conduit 852 are disposed at or near the treatment site (e.g., within an aneurysm sac). The occlusive member 102 can be deployed (e.g., by retracting a surrounding elongate member 108 to allow the occlusive member 102 to self-expand) and the embolic element 230 may be conveyed through the lumen 854 of the conduit and to the treatment site. Following deployment of the occlusive member 102 and delivery of the embolic element 230, the occlusive member 102 can be electrolytically detached from the core wire 812, and the system 1000 can be proximally retracted, leaving the occlusive member 102 and embolic element 230 in position within the aneurysm or other treatment site.

### B. Detachment via Electrolytically Corrodible Conduit

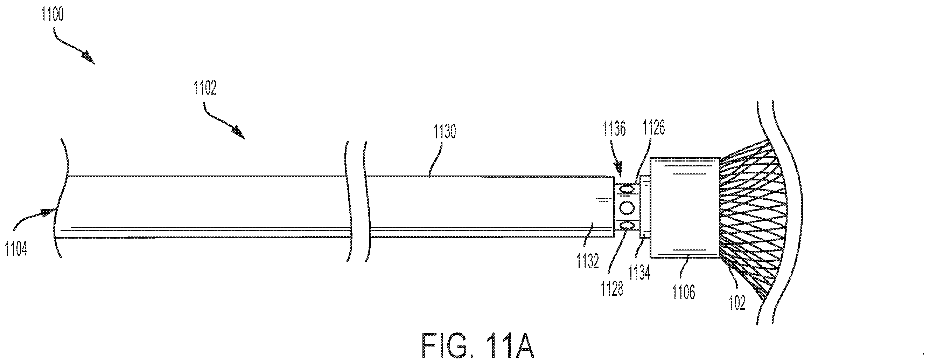

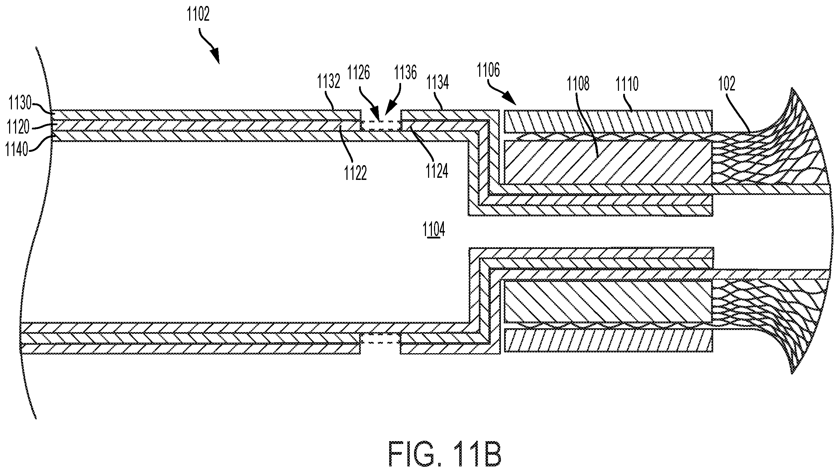

FIG. 11A shows a schematic side view of a treatment system 1100, and FIG. 11B shows a side cross-sectional view of a distal portion of the treatment system 1100 shown in FIG. 11A. As described in more detail below, the treatment system 1100 can include a conduit assembly 1102 that is releasably coupled to the occlusive member 102. In operation, the treatment system 1100 facilitates placement of the occlusive member 102 at the treatment site and utilizes electrolytic detachment to release the occlusive member 102 from the conduit assembly 1102. As described in more detail below, a distal portion of the conduit assembly 1102 may remain in place alongside the occlusive member 102 following electrolytic detachment. Furthermore, the conduit assembly 1102 can facilitate introduction of an embolic element 230 (FIGS. 2-3G) therethrough for placement at the treatment site (e.g., within an aneurysm sac accompanying the occlusive member 102).

Although several examples refer to the use of electrolytic detachment, in various embodiments other techniques can be used to sever a conduit and release the occlusive member 102. For example, instead of or in addition to electrolytic detachment, embodiments of the present technology may utilize thermal detachment, mechanical detachment, chemical detachment, or any other suitable detachment techniques.

Referring to FIGS. 11A and 11B together, the conduit assembly 1102 can take the form of an elongated tubular member defining a lumen 1104 therein. The conduit assembly 1102 can be coupled to a proximal hub 1106 of the occlusive member 102, such that the lumen 1104 extends distally beyond the proximal hub 1106. As shown in FIG. 11B, an inner band 1108 of the hub 1106 circumferentially surrounds a portion of the conduit assembly 1102. An outer band 1110 surrounds the inner band 1108, such that proximal portions of the layers of the occlusive member 102 are grasped between the inner and outer bands 1108, 1110 of the hub 1106. Such bands can be made of any suitable material, for example being polymeric or metallic, and optionally may be radiopaque to facilitate visualization of the system 1100 as it advanced through the vasculature. The bands can be crimped, with or without an adhesive or weld, to secure them in place. In operation, an embolic element can be introduced via the lumen 1104 into the treatment site (e.g., within an aneurysm sac) and adjacent the occlusive member 102. In some examples, the inner band 1108 can have an inner diameter of about 0.508 mm (0.020 inches), and the outer band 1110 can have an outer diameter of about 0.584 mm (0.023 inches).

In various embodiments, the conduit assembly 1102 can include a single tubular member or a plurality of tubular members arranged coaxially. Moreover, any one of the tubular members can be monolithic or can be formed of multiple separate components joined together. Additionally or alternatively, some or all of the tubular member(s) can include one or more coatings along some or all of their respective lengths. In some embodiments, one or more of the tubular members can be slidably moveable with respect to other tubular members. Alternatively or additionally, one or more of the tubular members can be fixed (e.g., non-slidably coupled) with respect to the other tubular members.

In the embodiment illustrated in FIGS. 11A-11B, the conduit assembly 1102 includes a conduit 1120 which takes the form of an elongated tubular member. An outer sheath 1130 extends along a radially outer surface of the conduit 120, and an inner liner 1140 extends along a radially inner surface of the conduit 1120. As described in more detail below, the conduit 1120 can include a detachment zone 1126 configured to be electrolytically corroded when current is supplied to the conduit 1120. The outer sheath 1130 and/or the inner liner 1140 can be electrically insulative such that current carried by the conduit 1120 is confined to the conduit 1120 and focused at the detachment zone 1126. When in the presence of an electrolytic medium, such as blood, current passes from the conduit 1120 to the surrounding media through the detachment zone 1126.

In various embodiments, the conduit assembly 1102 can have a length sufficient to permit the occlusive member 102 to be positioned at an intravascular treatment site (e.g., within an aneurysm sac) while a proximal end of the conduit assembly 1102 extends outside the patient's body. For example, the conduit assembly 1102 can have a length of greater than about 127cm (50 inches), 152.4cm (60 inches), 177.8cm (70 inches), or 203.2cm (80 inches). The conduit assembly 1102 can have an outer diameter suitable to permit the assembly 1102 to be slidably advanced through a delivery catheter. For example, the conduit assembly 1102 can have an outer diameter of less than about 0.686 mm (0.027 inches), less than about 0.533 mm (0.021 inches), or less than about 0.432 mm (0.017 inches).

In the example shown in FIGS. 11A-11B, the conduit assembly 1102 extends through the hub 1106 of the occlusive member 102, with a stepped down diameter at the hub 1106 resulting in a narrower lumen 1104 in a distal portion of the conduit assembly 1102 that extends through the hub 1106 and distal to the hub 1106. In some embodiments, this stepped-down diameter can result from crimping the hub 1106 over the conduit assembly 1102. In other embodiments, however, the conduit assembly 1102 need not have such a stepped-down inner and/or outer diameter. For example, the conduit assembly 1102 can have an outer diameter and/or an inner diameter that is substantially constant along its length, or that tapers gradually along some or all of its length.

As noted above, the conduit assembly 1102 includes a conduit 1120, which can be radially disposed between an outer sheath 1130 and an inner liner 1140. The conduit 1120 includes a proximal portion 1122, a distal portion 1124, and a detachment zone 1126 disposed axially between the proximal portion 1122 and the distal portion 1124. In some embodiments, the conduit 1120 can be an electrically conductive tubular member, for example a hypotube, catheter, or other suitable tubular member. In some embodiments, a portion of the conduit 1120, including the detachment zone 1126, can be coated with a conductive material, such as carbon, gold, platinum, tantalum, combinations thereof, and the like. One or more metallic coatings can be applied using known plating techniques. In various embodiments, the conduit 1120 can have cuts (e.g., a spiral cut, a groove, etc.) along at least a portion of its length to achieve the desired mechanical properties (e.g., column strength, flexibility, kink-resistance, etc.).

The conduit 1120 can be dimensioned to facilitate intravascular advancement to the treatment site and to accommodate a lumen 1104 sufficient to permit advancement of embolic element(s) therethrough. In some embodiments, the conduit 1120 can have a wall thickness of between about 0.0127 mm (0.0005 inches) and about 0.0381 mm (0.0015 inches), or about 0.0254 mm (0.001 inches) in some examples. The conduit 1120 can have an outer diameter in the proximal portion of less than about 0.6858 mm (0.027 inches), less than about 0.5334 mm (0.021 inches), or less than about (0.4318 mm) 0.017 inches. Additionally or alternatively, the conduit 1120 can have an inner diameter of less than about 0.3810 mm (0.015 inches), less than about 0.3048 mm (0.012 inches), less than about 0.2540 mm (0.010 inches), or less than about 0.2032 mm (0.008 inches). As shown in FIG. 11B, the conduit 1120 can have a stepped-down diameter where the conduit 1120 passes through the hub 1106. For example, the conduit 1120 can have an outer diameter of about 0.4064 mm (0.016 inches) proximal to the hub 1106, and an outer diameter of about 0.3556 mm (0.014 inches) within the hub 1106. This reduced diameter can be achieved by crimping the bands of the hub 1106 over the conduit 1120, or by forming the conduit 1120 with a stepped-down profile prior to coupling the conduit 1120 to the hub 1106.

The conduit 1120, including the detachment zone 1126, can include one or more of the following materials: ceramic materials, plastics, base metals or alloys thereof, for example stainless steel or nitinol. Some of the most suitable material combinations for forming the electrolytically corrodible points can include one or more of the following: stainless steels, preferably of the type AISI 301, 304, 316, or subgroups thereof; Ti or TiNi alloys; Co-based alloys; noble metals; or noble metal alloys, such as Pt, Pt metals, Pt alloys, Au alloys, or Sn alloys. Further, ceramic materials and plastics employed for forming the medical device can be electrically conductive.

In some embodiments, the detachment zone 1126 can include features to facilitate electrolytic severability, such as features configured to reduce a time that current must be supplied to the conduit 1120 before the conduit is severed at the detachment zone 1126. In some embodiments, the detachment zone 1126 can include a sidewall having one or more openings 1128 formed therein, which can take the form of one or more windows, slits, apertures, holes, or other such features. The openings 1128 can both increase the surface-area-to-volume ratio at the detachment zone 1126, and can also reduce the overall amount of material forming the sidewall of the conduit 1120 at the detachment zone 1126. As a result, the sidewall material of the conduit 1120 at the detachment zone 1126 may be more readily electrolytically corroded when current is supplied to the conduit 1120 and the detachment zone 1126 is exposed to an electrolytic medium such as blood. Additionally or alternatively to the openings 1128, the detachment zone 1126 can include a reduced sidewall thickness of the conduit 1120, and/or otherwise provide a lower material density than the proximal and distal portions 1122, 1124 of the conduit 1120. In some embodiments, the detachment zone 1126 can be surface treated (e.g., using laser or chemical treatment) to create a microstructure at the detachment zone 1126 that differs from that of the proximal and distal portions 1122, 1124 of the conduit 1120 to facilitate electrolytic detachment. For example, the detachment zone 1126 can have a microstructure having a lower crystallinity than each of the conduit proximal portion 1122 and conduit distal portion 1124. As another example, the detachment zone 1126 can have a microstructure that is more amorphous than each of the conduit proximal portion 1122 and conduit distal portion 1124.

According to some embodiments, portions of the conduit 1120 can be covered with an electrically insulative material. For example, a sheath 1130 that is made of or includes an electrically insulative material can extend over a radially outer surface of the conduit 1120 along at least a portion of the length of the conduit 1120. For example, the sheath 1130 can include a proximal portion 1132 that circumferentially surrounds an outer surface of the conduit proximal portion 1122. The sheath 1130 can also include a distal portion 1134 that circumferentially surrounds an outer surface of the conduit distal portion 1124. A void or gap 1136 can separate the sheath proximal and distal portions 1132, 1134. In some embodiments, the sheath proximal and distal portions 1132, 1134 can be discrete members that are not connected to one another, while in other embodiments the proximal and distal portions 1132, 1134 may be connected across the gap 1136, for example via connecting strands of material.

The sheath 1130 can be fully or partially made of an electrically nonconductive or insulative polymer, such as polyimide, polypropylene, polyolefins, combinations thereof, and the like. In some embodiments, the sheath 1130 takes the form of an extruded polymeric tube (e.g., PTFE), and the sheath 1130 extends distally beyond the hub 1106 and distally beyond a distal end of the conduit 1120. Accordingly, in some embodiments, the sheath 1130 can define the distal opening of the conduit assembly 1102. In some embodiments, the distal end of the sheath 1130 is disposed adjacent or distal to a distal end of the occlusive member 102 when the occlusive member 102 is in its expanded state. According to some embodiments, the distal end of the sheath 1130 is disposed adjacent or distal to a distal end of the occlusive member 102 when the occlusive member 102 is in its low-profile state.

The sheath 1130 can be dimensioned to facilitate intravascular advancement to the treatment site and to accommodate a lumen 1104 sufficient to permit advancement of embolic element(s) therethrough. In some embodiments, the sheath 1130 can have a wall thickness of between about 0.0127 mm (0.0005 inches) and about 0.0508 mm (0.002 inches), or about 0.0381 mm (0.0015 inches) in some examples. The sheath 1130 can have an outer diameter in the proximal portion of less than about 0.6858 mm (0.027 inches), less than about 0.5334 mm (0.021 inches), less than about 0.4318 mm (0.017 inches), or less than about 0.3810 mm (0.015 inches). Additionally or alternatively, the sheath 1130 can have an inner diameter of less than about 0.3810 mm (0.015 inches), less than about 0.3048 mm (0.012 inches), less than about 0.2540 mm (0.010 inches), or less than about 0.2032 mm (0.008 inches). As shown in FIG. 11B, the sheath 1130 can have a stepped-down diameter, similar to that described above with respect to the conduit 1120.

According to some embodiments, a gap 1136 between the sheath proximal and distal portions 1132, 1134 leaves exposed the detachment zone 1126 of the underlying conduit 1120. When in contact with a body fluid, such as blood, the fluid serves as an electrolyte allowing current to be focused on the non-covered detachment zone 1126. The sheath proximal and distal portions 1122, 1124 prevent exposure of the conduit proximal portion 1122 and the conduit distal portion 1124 to the fluid. Accordingly, electrical energy conducted along the conduit 1120 is concentrated at the detachment zone 1126, thereby reducing the time required to erode away the detachment zone 1126. The sheath proximal and distal portions 1132, 1134 can be slidably disposed over, over-molded, co-extruded, sprayed on, or dip-coated with respect to the conduit 1120.

The gap 1136 between the sheath proximal portion 1132 and the sheath distal portion 1134 can be dimensioned so as to achieve the desired exposure of the underlying detachment zone 1126. According to some embodiments, the gap 1136 can be as small as 0.0127 mm (0.0005 inches) and as large as 2.5400 mm (0.1 inches) or longer. According to some embodiments, lengths of detachment zone 1126 can be greater than 0.1270 mm (0.005 inches) and/or less than 0.2540 mm (0.010 inches) to provide sufficient exposure to achieve detachment times of less than 30 seconds.

According to some embodiments, the sheath distal portion 1134 is disposed radially between the distal portion 1124 of the conduit 1120 and the hub 1106 of the occlusive member 102. As shown in FIG. 11B, the inner band 1108 of the hub 1106 circumferentially surrounds and contacts the distal portion 1134 of the sheath 1130. The insulative sheath distal portion 1134 can electrically isolate the occlusive member 102 from an electrical charge conducted along a length of the conduit 1120. A proximal end of the sheath distal portion 1134 may be positioned proximal to the hub 1106, and a distal end of the sheath distal portion 1134 may be positioned distal to the hub 1106. Alternatively, the proximal end of the sheath distal portion 1134 may be coterminous with a proximal end of the hub 1106, and/or a distal end of the sheath distal portion 1134 may be coterminous with a distal end of the hub 1106.

As noted above, an inner liner 1140 can be disposed radially inwardly of the conduit 1120. The liner 1140 can be an elongate tubular member and can be made of an electrically insulative material. In some embodiments, the liner 1140 can have an inner surface defining the lumen 1104 along at least a portion of the length of the conduit assembly 1102. Accordingly, the inner surface of the liner 1140 can be continuous and uninterrupted along its length, such that liquid embolic material passing therethrough is contained within the lumen 1104 until it reaches a distal end of the liner 1140. In particular, the liner 1140 can provide a continuous and uninterrupted surface along the detachment zone 1126 of the conduit 1120, such that any embolic element(s) cannot pass from within the lumen 1104 through the openings 1128 in the conduit 1120 at the detachment zone 1126.

In various embodiments, the liner 1140 can extend distally to be coterminous with the conduit 1120 (e.g., at or adjacent a distal end of the hub 1106), or alternatively the liner 1140 can extend distally beyond the hub 1106 and/or distally beyond a distal end of the conduit 1120. The liner 1140 can be made of or coated with a lubricious material to facilitate advancement of embolic element(s) therethrough. In some embodiments, the liner 1140 takes the form of an extruded polymeric tube (e.g., PTFE) or other suitable electrically insulative material. Additionally or alternatively, the inner liner 1140 can be co-extruded, sprayed on, or dip-coated with respect to the conduit 1120.

The liner 1140 can be dimensioned to facilitate intravascular advancement to the treatment site and to accommodate a lumen 1104 sufficient to permit advancement of embolic element(s) therethrough. In some embodiments, the liner 1140 can have a wall thickness of between about 0.0127 mm (0.0005 inches) and about 0.0381 mm (0.0015 inches), or about 0.0254 mm (0.001 inches) in some examples. The liner 1140 can have an outer diameter in the proximal portion of less than about 0.6858 mm (0.027 inches), less than about 0.5334 mm (0.021 inches), or less than about 0.4318 mm (0.017 inches). Additionally or alternatively, the liner 1140 can have an inner diameter of less than about 0.3810 mm (0.015 inches), less than about 0.3048 mm (0.012 inches), less than about 0.2540 mm (0.010 inches), or less than about 0.2032mm (0.008 inches). As shown in FIG. 11B, the liner 1140 can have a stepped-down diameter where the liner 1140 passes through the hub 1106, similar to that of the conduit 1120 and sheath 1130 described above.

In some embodiments, an embolic element can be delivered through the lumen 1104 of the conduit assembly 1102. The lumen 1104 can terminate in a distal opening (not shown). As noted above, in some embodiments, the conduit assembly 1102 can include an elongate flexible tubular member, for example a catheter, hypotube, polymer tube, etc. The lumen 1104 can be coated with a lubricious material or lining to facilitate advancement of embolic element(s) therethrough. In some embodiments, the conduit assembly 1102 is dimensioned such that the distal opening is disposed adjacent to, completely distal of, or at least partially distal of the occlusive member 102 while the occlusive member 102 is in the unexpanded state. The conduit assembly 1102 can be dimensioned and configured such that the distal opening is disposed at distal to the hub 1106 of the occlusive member 102, such that embolic element(s) delivered therethrough can be delivered to a region adjacent or distal of the occlusive member 102.

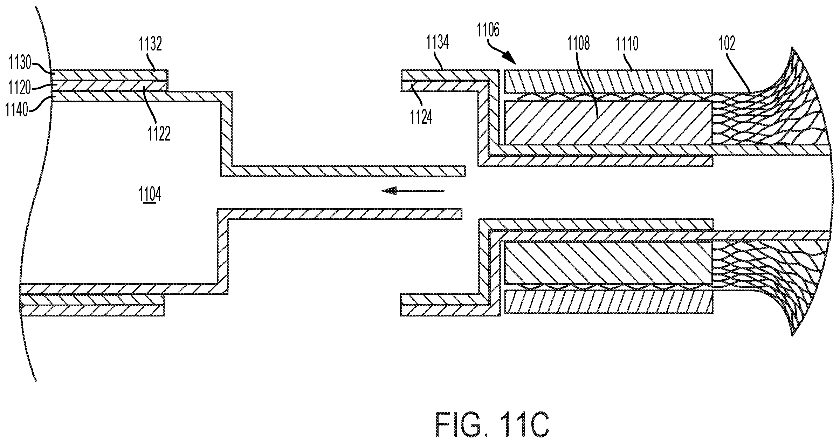

FIG. 11C illustrates the treatment system 1100 with the conduit assembly 1102 partially retracted following electrolytic severance of the conduit 1120 at the detachment zone 1126. As illustrated, the sheath distal portion 1122 and the conduit distal portion 1124 can remain coupled to the hub 1106 of the occlusive member 102, while the sheath proximal portion 1122, conduit distal portion 1122, and the liner 1140 are retracted proximally. According to some embodiments, the conduit assembly 1120 can be retracted through a surrounding catheter and removed from the body completely.

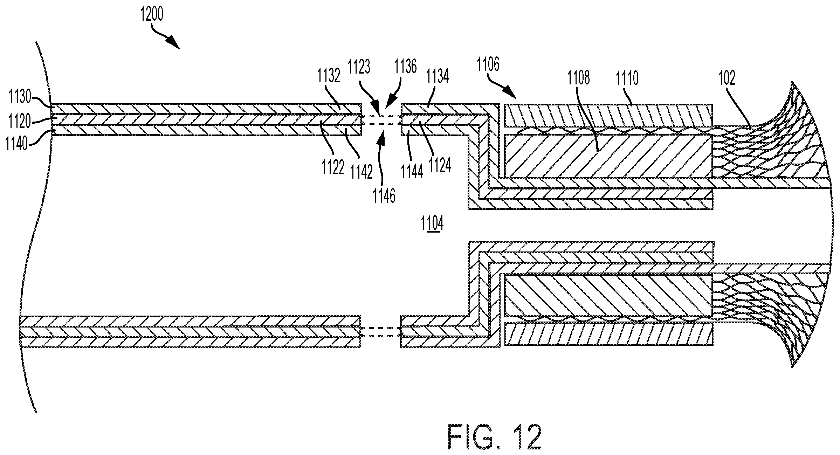

FIG. 12 shows a schematic side view of another embodiment of a treatment system 1200 in accordance with aspects of the present technology. The treatment system 1200 can include several features that are generally similar to those of FIGS. 11A-11C described above. However, in the treatment system 1200 shown in FIG. 12, the liner 1140 includes a proximal portion 1142 and a distal portion 1124 that are spaced apart from one another by a gap 1146 that is axially aligned with the detachment zone 1126. In this configuration, following electrolytic severance of the conduit 1120 at the detachment zone 1126, the liner distal portion 1144 may remain in place along with the occlusive member 102, the conduit distal portion 1124, and the sheath distal portion 1134. As such, the liner proximal portion 1142 can be retracted along with the conduit proximal portion 1122 and sheath proximal portion 1132.

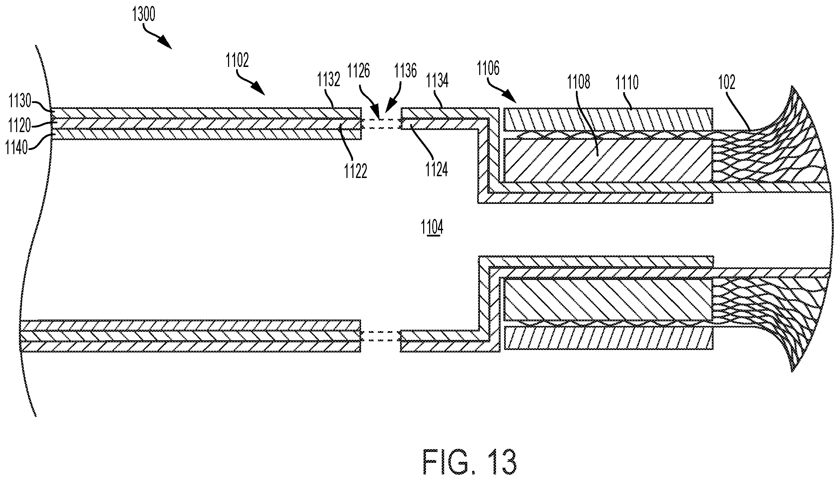

FIG. 13 shows a schematic side view of another embodiment of a treatment system 1300 in accordance with aspects of the present technology. The treatment system 1300 can include several features that are generally similar to those of FIGS. 11A-12 described above. However, in the treatment system 1300 shown in FIG. 6, the liner 1140 terminates at or proximal to the detachment zone 1126. In this configuration, the lumen 1104 is defined by the liner 1140 along a portion of the length of the treatment system 1300, and is defined by the inner surface of the conduit 1120 along a distal portion of the conduit assembly 1102.

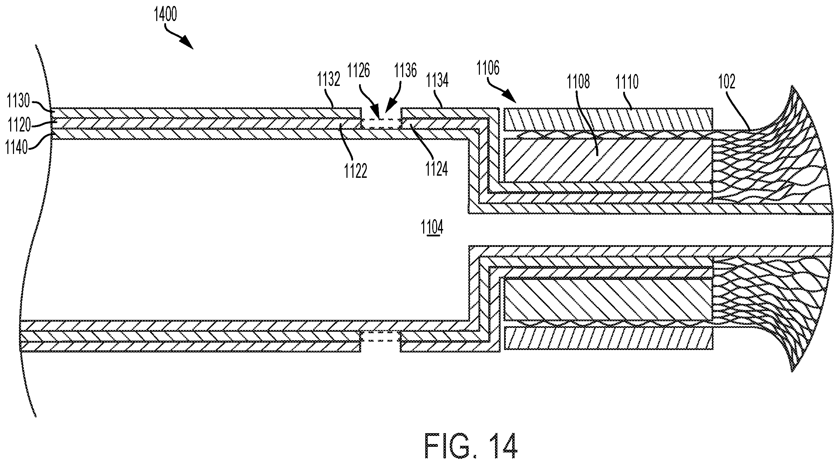

FIG. 14 shows a schematic side view of another embodiment of a treatment system 1400 in accordance with aspects of the present technology. The treatment system 1400 can include several features that are generally similar to those of FIGS. 11A-13 described above. However, in the treatment system 1400 shown in FIG. 14, the sheath 1130 terminates distally at or adjacent a distal end of the hub 1106. Accordingly, the sheath 1130 and the conduit 1120 can be substantially co-terminal. Meanwhile, the inner liner 1140 can extend distally beyond the hub 1106 and beyond the distal ends of the conduit 1120 and sheath 1130. Accordingly, in this configuration, the lumen 1104 is defined along its entire length by the inner surface of the liner 1140. Following severance of the conduit 1120 at the detachment zone 1126, the liner 1140 can be proximally retracted along with the conduit proximal portion 1122 and the sheath proximal portion 1132. As such, following this proximal retraction, there remains no tubular member extending into an interior of the occlusive member 102, in contrast to the embodiments described above with respect to FIGS. 11A-11C. This arrangement may be beneficial if it is desirable to remove any tubular element from within the sac of the aneurysm following deployment of the occlusive member 102 and any embolic element(s).

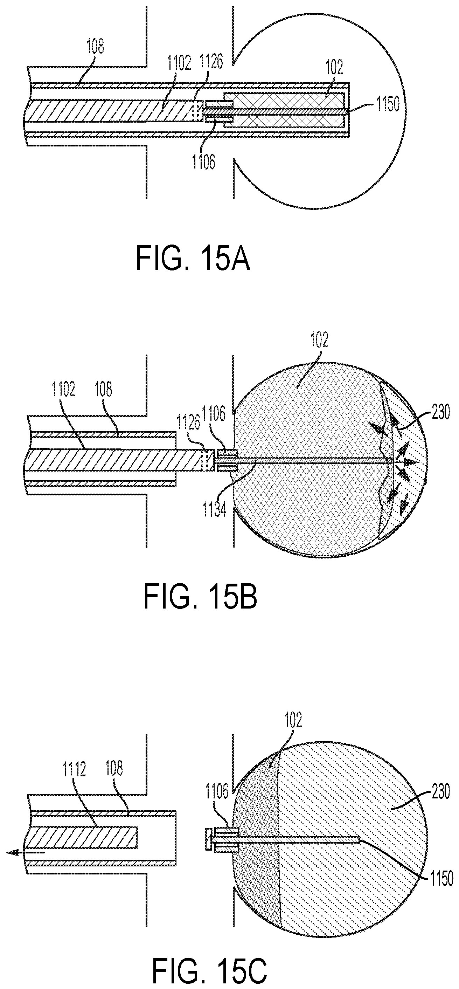

FIGS. 15A-15C illustrate delivery of an occlusive member 102 and embolic element 230 to a treatment site within an aneurysm sac. As shown in FIG. 15A, the treatment system 1100 can be positioned within a second elongate shaft 108 (e.g., a microcatheter) for intravascular advancement until the microcatheter is at or adjacent to the aneurysm sac. In the illustrated embodiment, the distal end of the second elongate shaft 108 extends within the aneurysm sac, however in other embodiments the distal end of the second elongate shaft 108 can be positioned at the neck of the aneurysm or proximal to the neck of the aneurysm.

In the position shown in FIG. 15A, the system 1100 has been advanced within the elongate shaft 108 such that the occlusive member 102 remains in a constrained, low-profile configuration within the shaft 108 while at least a portion of the conduit assembly 1102 extends adjacent to the occlusive member 102 and within the shaft 108. In various embodiments, the shaft 108 can have an inner diameter of about 0.432 mm (0.017 inches) or less, about and 0.533mm (0.021 inches) or less, or about 0.686mm (0.027 inches) or less.

As shown in FIG. 15B, once the distal opening 1150 of the conduit assembly 1102 is positioned at or near the treatment site (e.g., within the aneurysm sac), the elongate shaft 108 can be retracted, thereby deploying the occlusive member 102 within the aneurysm sac (e.g., allowing the occlusive member 102 to self-expand). In this position, the embolic element 230 can be advanced through the conduit assembly 1102 and into the aneurysm to a region distal to the occlusive member 102. In the case of a fluid or gel, a syringe or other injector may be used to urge the embolic element 230 through the lumen 1104. In the case of microcoils or other structural embolic element(s), a delivery wire or other suitable mechanism may be slidably advanced through the lumen 1104 of the conduit assembly 1102 to position the embolic element 230 into the aneurysm sac.

As described previously with respect to FIGS. 3A-3G, introduction of the embolic element 230 can cause the occlusive member 102 to deform, for example to at least partially fold in on itself to provide for increased protection in a neck region of the aneurysm. Once the embolic element 230 been delivered and the occlusive member 102 has deformed, the occlusive member 102 can be severed from the conduit assembly 1102 as described above. For example, a power supply or other current source can be used to generate current through the conduit 1120, resulting in electrolytic corrosion of the conduit 1120 at the detachment zone 1126.

As shown in FIG. 15C, after the occlusive member 102 is released via electrolytic corrosion of the detachment zone 1126, the conduit assembly 1102 can be proximally retracted while the occlusive member 102 and the embolic element 230 remain positioned within the aneurysm. As the conduit assembly 1102 is retracted, the distal portion of the conduit assembly (e.g., the distal portion 1134 of the sheath 1130 and/or the distal portion 1124 of the conduit 1120) can remain within the aneurysm and coupled to the occlusive member 102.

### Conclusion

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A treatment system (800) comprising:
an electrolytically corrodible core wire (812) having a proximal portion (814), a distal portion (816), and a detachment zone between the proximal portion (814) and the distal portion (816);
an occlusive member (102) having a proximal hub coupled to the core wire distal portion (816), the occlusive member (102) configured to be positioned at or adjacent to a treatment site; and wherein
the treatment system further includes:
a conduit (852) extending along at least a portion of the core wire (812), the conduit (852) having a lumen (854) configured to pass an embolic element therethrough,
wherein a distal portion of the conduit (852) is configured to be disposed at or adjacent the treatment site alongside the occlusive member (102),
wherein the treatment site comprises an aneurysm sac, and wherein the occlusive member (102) is configured to be disposed within the aneurysm sac and a distal portion of the conduit is configured to be disposed within the aneurysm sac for delivery of the embolic element to within the aneurysm sac,
**characterised in that** the conduit (852) distal portion (860) has a smaller cross-sectional dimension than a proximal portion (858) of the conduit (852).

2. The treatment system (800) of claim 1, wherein the conduit (852) comprises a flexible tubular member.

3. The treatment system (800) of claim 1 or claim 2, further comprising a stylet (864) configured to be removably disposed within the lumen (854) of the conduit (852).

4. The treatment system (800) of any preceding claim, wherein the conduit (852) is coupled to the core wire (812) such that the two cannot slide relative to one another.

5. The treatment system (800) of any preceding claim, wherein the occlusive member (102) comprises an expandable mesh having a constrained state for delivery to an aneurysm and an expanded state in which at least a portion of the mesh is configured to be disposed across a neck of the aneurysm.

## Patentansprüche

1. Behandlungssystem (800), umfassend:
einen elektrolytisch korrodierbaren Kerndraht (812), der einen proximalen Abschnitt (814), einen distalen Abschnitt (816) und eine Ablösungszone zwischen dem proximalen Abschnitt (814) und dem distalen Abschnitt (816) aufweist;
ein Verschlusselement (102), das eine proximale Nabe aufweist, die an den distalen Abschnitt (816) des Kerndrahts gekoppelt ist, wobei das Verschlusselement (102) konfiguriert ist, um an oder angrenzend an eine Behandlungsstelle positioniert zu werden; und wobei das Behandlungssystem ferner einschließt:
eine Leitung (852), die sich entlang mindestens eines Abschnitts des Kerndrahts (812) erstreckt, wobei die Leitung (852) ein Lumen (854) aufweist, das konfiguriert ist, um ein embolisches Element hindurchzuleiten,
wobei ein distaler Abschnitt der Leitung (852) konfiguriert ist, um an oder angrenzend an die Behandlungsstelle neben dem Verschlusselement (102) angeordnet zu werden,
wobei die Behandlungsstelle einen Aneurysmasack umfasst, und wobei das Verschlusselement (102) konfiguriert ist, um innerhalb des Aneurysmasacks angeordnet zu werden, und ein distaler Abschnitt der Leitung konfiguriert ist, um innerhalb des Aneurysmasacks angeordnet zu werden zur Abgabe des embolischen Elements in den Aneurysmasack,
**dadurch gekennzeichnet, dass**
der distale Abschnitt (860) der Leitung (852) eine kleinere Querschnittsabmessung aufweist als ein proximaler Abschnitt (858) der Leitung (852).

2. Behandlungssystem (800) nach Anspruch 1, wobei die Leitung (852) ein flexibles rohrförmiges Element umfasst.

3. Behandlungssystem (800) nach Anspruch 1 oder Anspruch 2, ferner umfassend einen Mandrin (864), der konfiguriert ist, um entfernbar innerhalb des Lumens (854) der Leitung (852) angeordnet zu werden.

4. Behandlungssystem (800) nach einem der vorstehenden Ansprüche, wobei die Leitung (852) an den Kerndraht (812) gekoppelt ist, sodass die beiden nicht relativ zueinander gleiten können.

5. Behandlungssystem (800) nach einem der vorstehenden Ansprüche, wobei das Verschlusselement (102) ein ausdehnbares Netz umfasst, das einen eingeschränkten Zustand für die Abgabe an ein Aneurysma und einen ausgedehnten Zustand aufweist, in dem mindestens ein Abschnitt des Netzes konfiguriert ist, um über einem Hals des Aneurysmas angeordnet zu werden.

## Revendications

1. Système de traitement (800) comprenant :
un fil central pouvant être corrodé par voie électrolytique (812) ayant une partie proximale (814), une partie distale (816) et une zone de détachement entre la partie proximale (814) et la partie distale (816) ;
un élément occlusif (102) ayant un moyeu proximal accouplé à la partie distale de fil central (816), l'élément occlusif (102) étant conçu pour être positionné au niveau ou à proximité d'un site de traitement ; et dans lequel le système de traitement comporte en outre :
un conduit (852) s'étendant le long d'au moins une partie du fil central (812), le conduit (852) ayant une lumière (854) conçue pour faire passer un élément d'embolisation à travers celui-ci,
dans lequel une partie distale du conduit (852) est conçue pour être disposée au niveau du site de traitement ou adjacent à côté de l'élément occlusif (102),
dans lequel le site de traitement comprend un sac d'anévrisme, et dans lequel l'élément occlusif (102) est conçu pour être disposé à l'intérieur du sac d'anévrisme et une partie distale du conduit est conçue pour être disposée à l'intérieur du sac d'anévrisme pour l'administration de l'élément embolique à l'intérieur du sac d'anévrisme,
**caractérisé en ce que**
la partie distale (860) du conduit (852) a une dimension de section transversale plus petite qu'une partie proximale (858) du conduit (852).

2. Système de traitement (800) selon la revendication 1, dans lequel le conduit (852) comprend un élément tubulaire flexible.

3. Système de traitement (800) selon la revendication 1 ou la revendication 2, comprenant en outre un stylet (864) conçu pour être disposé de manière amovible à l'intérieur de la lumière (854) du conduit (852).

4. Système de traitement (800) selon l'une quelconque revendication précédente, dans lequel le conduit (852) est accouplé au fil central (812) de telle sorte que les deux ne peuvent pas glisser l'un par rapport à l'autre.

5. Système de traitement (800) selon l'une quelconque revendication précédente, dans lequel l'élément occlusif (102) comprend un maillage extensible ayant un état contraint pour l'administration dans un anévrisme et un état déployé dans lequel au moins une partie du maillage est conçue pour être disposée en travers d'un col de l'anévrisme.
